# EUROPEAN PATENT SPECIFICATION

(11) **EP 1 781 257 B1**
(45) Date of publication and mention of the grant of the patent: **19.12.2018**
(21) Application number: 05857913.7
(22) Date of filing: 15.08.2005
(51) Int. Cl.: A61K 9/16, A61K 9/20, A61K 9/48, A61K 47/12

(54) **PHARMACEUTICAL FORMULATIONS CONTAINING MICROPARTICLES OR NANOPARTICLES OF A DELIVERY AGENT**
PHARMAZEUTISCHE FORMULIERUNGEN MIT MIKROPARTIKELN ODER NANOPARTIKELN EINES ZUFUHRMITTELS
FORMULATIONS PHARMACEUTIQUES CONTENANT DES MICROPARTICULES OU DES NANOPARTICULES D'UN AGENT D'ADMINISTRATION

(30) Priority: 13.08.2004 US 601258 P; 23.09.2004 US 612810 P
(43) Date of publication of application: 09.05.2007
(73) Proprietor: Emisphere Technologies, Inc., Roseland, NJ 07068 (US)
(72) Inventor: MAJURU, Shingai, Emisphere Technologies Inc., Tarrytown, NY 10591 (US); LIU, Puchun, Emisphere Technologies, Inc., Tarrytown, NY 10591 (US); DINH, Steven Emisphere Technologies, Inc., Tarrytown, NY 10591 (US); LIAO, Jun, Emisphere Technologies, Inc., Tarrytown, NY 10591 (US); LEE, Jongbin, Emisphere Technologies, Inc., Tarrytown, NY 10591 (US); ARBIT, Ehud, Emisphere Technologies, Inc., Tarrytown, NY 10591 (US); DHOOT, Nikhil, Dombivli (East) 421-203 (IN); LEVCHIK, Halina, Emisphere Technologies, Inc., Tarrytown, NY 10591 (US); HARRIS, Jamila, Emisphere Technologies, Inc., Tarrytown, NY 10591 (US); WANG, Nai Fang, Emisphere Technologies, Inc., Tarrytown, NY 10591 (US); KLEIN, George, F. Emisphere Technologies, Inc., Tarrytown, New York 10591 (US)
(74) Representative: Grünecker Patent- und Rechtsanwälte PartG mbB
(86) International application number: PCT/US2005/028991
(87) International publication number: WO 2006/124047

(56) References cited:
- WO-A1-94/23767
- WO-A1-99/11703
- WO-A2-2005/007139
- US-A- 5 534 488
- US-A- 5 650 386
- US-B1- 6 468 959
- US-B1- 6 491 903
- TRENKTROG T ET AL: "Enteric coated insulin pellets: Development, drug release and in vivo evaluation", EUROPEAN JOURNAL OF PHARMACEUTICAL SCIENCES, ELSEVIER, AMSTERDAM, NL, vol. 4, no. 6, 1 January 1996 (1996-01-01) , pages 323-329, XP002314437, ISSN: 0928-0987, DOI: 10.1016/0928-0987(95)00162-X
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

This invention relates to particles comprising a delivery agent and a biologically active agent, and to pharmaceutical formulations.

WO9911703 (A1) discloses cross-linked particles that are useful for delivery of pharmaceutical agents. The particles comprise at least one polymeric compound and a spacer compound, where the polymeric compound and the spacer each comprise reactive carboxyl, hydrazidyl, amino and/or thiol groups. The particles are cross-linked via covalent linkage of the reactive groups on the polymer and spacer respectively. Compositions comprising pharmaceutical agents contained within the particles are disclosed. Methods for preparing the particles, for encapsulating pharmaceutical agents within the particles, and for using the particles for controlled release of the pharmaceutical agent within the patient also are disclosed.

US6468959 (B1) discloses a plural dosage form for peptide pharmaceuticals comprising a matrix of gelatin or gelatin derivative having distributed therein the peptide pharmaceutical in particular insulin, as well as, pharmaceutically conventional carriers and additives. By selection of the appropriate gelatin the pharmaceutical is liberated in the small intestine or the large intestine so that it is not enzymatically degraded anymore by peptidases.

Enteric coated insulin pellets and their development and in vivo evaluation are disclosed in Trenktrog T. et al., European Journal of Pharmaceutical Sciences, Elsevier, Amstrerdam, NL, vol. 4, no. 6, pages 323 - 329.

WO9423767 (A1) discloses an oral drug delivery system, and in particular modified amino acids and modified amino acid derivatives for use as a delivery system of sensitive agents such as bioactive peptides. The modified amino acids and derivatives can form non-covalent mixtures with active biological agents and can releasably carry active agents. Modified amino acids can also form drug containing microspheres. These mixtures are suitable for oral administration of biologically active agents to animals. Methods for the preparation of such amino acids are also disclosed.

WO2005007139 (A2) discloses an oral, multiparticle pharmaceutical dosage form containing pellets, the size of which ranges from 50 to 2500 nm and which essentially consist of: a) an inner matrix layer containing an active substance which is a peptide or a protein, including the derivatives or conjugates thereof, and which is embedded in a matrix consisting of a polymer with mucoadhesive effect, and b) an outer film coating essentially consisting of an anionic polymer or copolymer, which can be optionally formulated with pharmaceutically conventional adjuvants, more particularly softening agents.

There is a continuing need for improved oral delivery systems for drugs, such as insulin.

The present invention provides particles comprising a delivery agent and a biologically active agent, wherein the particles have a median particle size of 250 to 425 micrometers and the delivery agent is a compound of Formula A, or a pharmaceutically acceptable salt thereof, 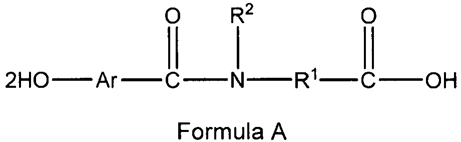 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

In another embodiment, the present invention provides a pharmaceutical formulation comprising particles having a median particle size of 250 to 425 micrometers, the particles comprising a delivery agent and a biologically active agent,
wherein the delivery agent is a compound of Formula A, or a pharmaceutically acceptable salt thereof, 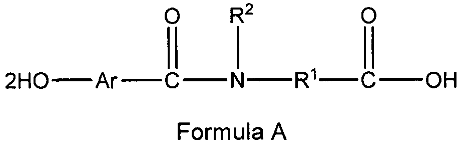 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

In another embodiment, the present invention provides a pharmaceutical formulation comprising a delivery agent and a biologically active agent, wherein the delivery agent is in the form of particles having a median particle size of 250 to 425 micrometers, and the delivery agent is a compound of Formula A, or a pharmaceutically acceptable salt thereof, 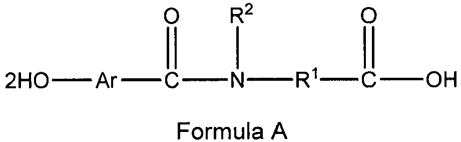 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁C₁₀ alkyl), or naphthyl(C₁C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

In another embodiment, the present invention provides a pharmaceutical formulation comprising a delivery agent and a biologically active agent, wherein the biologically active agent is in the form of particles having a median particle size of 250 to 425 micrometers, and the delivery agent is a compound of Formula A, or a pharmaceutically acceptable salt thereof, 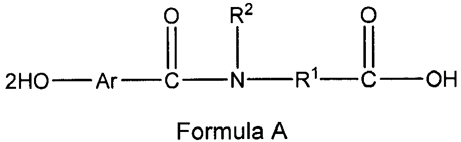 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁₋C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

In another embodiment, the present invention provides a solid dosage unit form comprising the pharmaceutical formulation of the invention.

In another embodiment, the present invention provides a pharmaceutical formulation of the invention for use in treating diabetes in a mammal in need thereof.

In another embodiment, the present invention provides a pharmaceutical formulation of the invention for use in treating impaired glucose tolerance, early stage diabetes, or late stage diabetes or achieving glucose homeostasis in a human in need thereof.

In another embodiment, the present invention provides a pharmaceutical formulation of the invention for use in treating a human diabetic patient, comprising orally administering the pharmaceutical formulation on a chronic basis.

Preferred embodiments are set forth in the subclaims.

Thus, the present invention relates to microparticles and/or nanoparticles for oral administration containing a delivery agent compound alone or a combination of a delivery agent compound and a biologically active agent. Formulations containing these particles (and, for particles containing only a delivery agent compound, and a biologically active agent) provide significantly greater bioavailability of the active agent with less variability than oral administration of a simple mixture of the delivery agent compound and active agent as a
powder, tablet, or capsule. Without being bound by any particular theory, it is believed that in at least some embodiments, this improvement may be due to (1) the small size of the micro- or nano-particles which permits them to pass from the stomach, through the pylorus (which typically has a diameter of 1000-2000 µm), to the small intestine, where particle dissolution and delivery agent-mediated drug absorption is believed to best occur, and (2) the intimate contact between the delivery agent compound and active agent in the particles which ensures that the delivery agent compound is present with the active agent at the site of absorption. Because the micro- and nano-particles freely pass through the pylorus into the small intestine, unlike a conventional tablet or capsule which must first become dissolved into particles sufficiently small to do so, variations caused by tablet disintegration and gastric transit modulated by gastric motility are minimized.

The particles can be in the form of fine granules or micro-beads (e.g., beads having a round/ball shape and a diameter of about 0.2 mm to about 2.0 mm). The micro-beads may be formed by compression. In one embodiment, the pharmaceutical formulation includes micro-beads containing a delivery agent compound, which are coated with an active agent, such as insulin or heparin. The micro-beads may have a diameter ranging from about 0.2 mm to 2.0 mm.

The particles may also include a mucoadhesive, such as a cellulose derivative (e.g., CMC sodium (available from Aqualon of Wilmington, DE)) or a polyacrylic acid (e.g., Carbopol™ available from B.F. Goodrich of Cleveland, OH). The mucoadhesive can (1) facilitate adhesion to mucosa (including in the gastrointestinal tract) thereby prolonging delivery agent-active agent contact with the mucosa, (2) stabilize and protect the active agent (e.g., in the case of insulin), and (3) increase the permeability of biomembranes (including mucosa) thereby improving delivery and increasing bioavailability of the active agent.

It has also been discovered that oral administration of insulin in conjunction with a delivery agent compound by solid oral dosage forms that do not degrade in the stomach, but do degrade in the intestine, provides significantly greater bioavailability of the insulin. Such solid oral dosage forms containing insulin or a different active agent provide greater bioavailability than forms that degrade in the stomach and forms that do not contain the delivery agent compound. Without being bound by any particular theory, it is believed that this improvement is due to the sensitivity of insulin and other active agents to degradation by enzymes or acid found in gastric fluid. Because the solid oral dosage forms do not degrade in the stomach, the insulin and other active agents are protected from degradation until they reach the intestine.

The present specification further discloses a pharmaceutical formulation (such as a solid oral dosage form) comprising a therapeutically effective amount of an active agent and a delivery agent, where the pharmaceutical formulation has a disintegration time of about 250 seconds to about 650 seconds when orally administered. In another embodiment, the disintegration time is about 350 to about 550 seconds when orally administered. In yet another embodiment, the disintegration time is greater than 60 seconds when orally administered. In yet another embodiment, the disintegration time is greater than 400 seconds when orally administered. Disintegration time can be determined in water at 37 ± 2°C using the method described in USP <701>. Disintegration times may range from about 1 second to as much as about 24 hours, or more, depending on many factors including the particular active agent(s), delivery agent compound(s), and excipients included in the pharmaceutical formulation.
rhe present specification further discloses a pharmaceutical formulation (such as a solid oral dosage form) comprising a therapeutically effective amount of an active agent and a delivery agent, where the solid oral dosage form does not substantially disintegrate or dissolve in the stomach, but does substantially disintegrate or dissolve in the intestine. In a preferred embodiment, the active agent is insulin. In another preferred embodiment, the active agent is an insulin derivative.
The present specification further discloses a pharmaceutical formulation that is a solid oral dosage form which is covered with an enteric coating to retard disintegration in the stomach. Enteric coatings include hydroxypropyl methylcellulose phthalate, hydroxypropyl methylcellulose acetate succinate, polyvinyl acetate phthalate, cellulose acetate trimellitate, cellulose acetate phthalate, poly(methacrylic acid-ethylacrylate), and poly(methacrylic acid-methyl methacrylate).

In yet another embodiment, the pharmaceutical formulations may be formulated to erode from the surface of the dosage form, rather than disintegrate.

The pharmaceutical formulations may include enzyme-inhibiting agents to prevent enzymatic degradation of active agents in the pharmaceutical formulation.

The delivery agent is a compound having the following structure or a salt thereof: 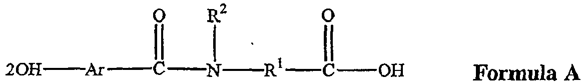 wherein
Ar is phenyl or naphthyl;
Ar is optionally substituted with one or more of -OH, halogen, C₁-C₄ alkyl, C₁-C₄ alkenyl, C₁-C₄ alkoxy. or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁-C₁₀ alkyl), or naphthyl(C₁-C₁₀ alkenyl);
R¹ is optionally substituted with C₁ to C₄ alkyl, C₂ to C₄ alkenyl, C₁ to C₄ alkoxy, C₁-C₄ haloalkoxy, -OH, -SH, -CO₂R⁸, or any combination thereof;
R² is hydrogen, C₁ to C₄ alkyl, or C₂ to C₄ alkenyl; and
R¹ is optionally interrupted by oxygen, nitrogen, sulfur or any combination thereof.

The term "2-OH-Ar" in formula A refers to a phenyl or naphthyl group having a hydroxyl group at the 2-position.

According to one embodiment, the compounds are not substituted with an amino group in the position alpha to the acid group.

Preferably, Ar is substituted with a halogen.

Preferably, R² is hydrogen.

Preferably, R¹ is unsubstituted.

Preferably, R¹ is not interrupted.

Preferably, R¹ is C₁₋₁₀, C₃₋₉, C₃₋₇, C₃, C₇, or C₉ alkyl. According to one embodiment, R¹ is not branched.

Preferred delivery agent compounds include N-(8-[2-hydroxybenzoyl]amino)caprylic acid (the free acid of SNAC), N-(10-[2-hydroxybenzoyl]amino)decanoic acid (the free acid of SNAC), 4-[(2-hydroxy-4-chlorobenzoyl)-amino]butanoic acid (the free acid of 4-CNAB), and salts thereof, and solvates and hydrates thereof. The salt can be, for example, a sodium salt, such as a monosodium (i.e., SNAC, SNAD, or 4-CNAB) or disodium salt.

The present specification further discloses a delivery agent that is a compound having the following structure or a salt thereof: 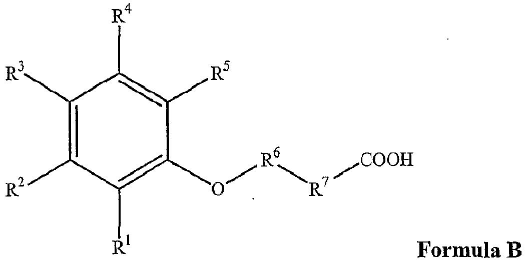 wherein
R¹, R², R³, and R⁴ are independently H, -OH, halogen, C₁-C₄ alkyl, C₂-C₄ alkenyl, C₁-C₄ alkoxy, -C(O)R⁸, -NO₂, -NR⁹R¹⁰, or -N⁺R⁹R¹⁰R¹¹ (R¹²)⁻;
R⁵ is H, -OH, -NO₂, halogen, -CF₃, - NR¹⁴R¹⁵, -N⁺R¹⁴R¹⁵R¹⁶ (R¹³ )⁻, amide, C₁-C₁₂ alkoxy, C₁-C₁₂ alkyl, C₂-C₁₂ alkenyl, carbamate, carbonate, urea, or -C(O)R¹⁸;
R⁵ is optionally substituted with halogen, -OH, -SH, or -COOH;
R⁵ is optionally interrupted by O, N, S, or -C(O)-;
R⁶ is a C₁-C₁₂ alkylene, C₂-C₁₂ alkenylene, or arylene;
R⁶ is optionally substituted with a C₁-C₄ alkyl, C₂-C₄ alkenyl, C₁-C₄ alkoxy, -OH,-SH, halogen, -NH₂, or -CO₁R⁸;
R⁶ is optionally interrupted by O or N;
R⁷ is a bond or arylene;
R⁷ is optionally substituted with -OH, halogen, -C(O)CH₃, -NR¹⁰R¹¹, or -N⁺R¹⁰R¹¹R¹² (R¹³)⁻;
R⁸ is H, C₁-C₄ alkyl, C₂-C₄ alkenyl, or -NH₂;
R⁹, R¹⁰, R¹¹, and R¹² are independently H or C₁-C₁₀ alkyl;
R¹³ is a halide, hydroxide, sulfate, tetrafluoroborate, or phosphate;
R¹⁴, R¹⁵, and R¹⁶ are independently H, C₁-C₁₀ alkyl, C₁-C₁₀ alkyl substituted with-COOH, C₂-C₁₂ alkenyl, C₂-C₁₂ alkenyl substituted with -COOH, or -C(O)R¹⁷;
R¹⁷ is -OH, C₁-C₁₀ alkyl, or C₂-C₁₂ alkenyl; and
R¹⁸ is H, C₁-C₆ alkyl, -OH, -NR¹⁴R¹⁵, or N⁺R¹⁴R¹⁵R¹⁶ (R¹³)⁻.
The present specification further discloses a delivery agent that is a compound having the following structure or a salt thereof: 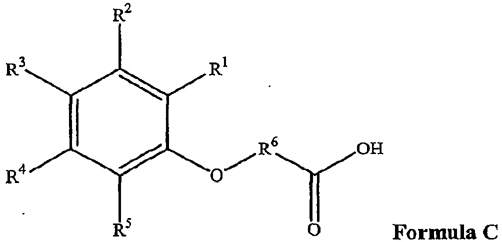 wherein
R¹, R², R³, R⁴ and R⁵ are independently H, -CN, -OH, -OCH₃, or halogen, at least one of R¹, R², R³, R⁴ and R⁵ being -CN; and
R⁶ is a C₁-C₁₂ linear or branched alkylene, alkenylene, arylene, alkyl(arylene) or aryl(alkylene).
The present specification further discloses a delivery agent that is a compound having the following structure or a. salt thereof: 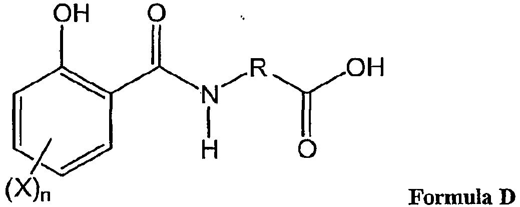 wherein
each occurrence of X is hydrogen, halogen, hydroxyl, or C₁-C₃ alkoxy,
R is substituted or unsubstituted C₁-C₃ alkylene or substituted or unsubstituted C₂-C₃ alkenylene, and
n is an integer from 1 to 4.
The present specification further discloses a delivery agent that is a compound having the following structure or a salt thereof: 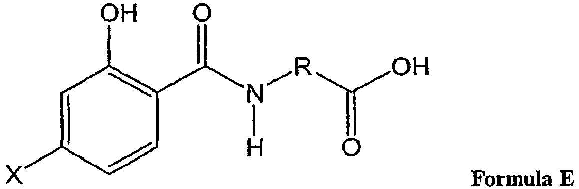 wherein
X is halogen, and R is substituted or unsubstituted C₁-C₃ alkylene or substituted or unsubstituted C₂-C₃ alkenylene.

Preferred delivery agent compounds include N-(8-[2-hydroxybenzoyl]-amino)caprylic acid, N-(10-[2-hydroxybenzoyl]-amino)decanoic acid, 8-(2-hydroxy-4-methoxybenzoylamino)octanoic acid, 8-(2-hydroxy-5-chlorobenzoylamino)-octanoic acid, 4-[(2-hydroxy-4-chlorobenzoyl)amino]butanoic acid, and pharmaceutically acceptable salts thereof. The pharmaceutical formulations of the present invention may include any of the aforementioned delivery agent compounds, or any other delivery agent compounds, alone or in combination with one or more additional delivery agent compounds.

Suitable active agents include proteins, polypeptides, peptides, hormones, polysaccharides, as well as synthetic, natural or recombinant sources thereof: growth hormones; growth hormone releasing hormones; growth hormone releasing factor, interferons; interleukin-1; interleukin-2; insulin, optionally having counter ions including zinc, sodium, calcium and ammonium; insulin-like growth factor; heparin; calcitonin; erythropoietin; atrial naturetic factor; antigens; monoclonal antibodies; somatostatin; protease inhibitors; adrenocorticotropin, gonadotropin releasing hormone; oxytocin; leutinizing-hormone-releasing-hormone; follicle stimulating hormone; glucocerebrosidase; thrombopoietin; filgrastim; prostaglandins; cyclosporin; vasopressin; cromolyn sodium; vancomycin; desferrioxamine; bisphosphonates; parathyroid hormone; anti-migraine agents; glucagon-like peptide 1 (GLP-1); antimicrobials; vitamins; and analogs, fragments, mimetics or polyethylene glycol (PEG)-modified derivatives of these compounds; or any combination thereof. Preferred active agents include insulin and heparin (including unfractionated heparin and low molecular weight heparin).

In one embodiment of the present invention, the active agent is insulin. The insulin-containing pharmacuetical formulations of the present invention may also include a second hypoglycemic agent, an inhibitor of renal glucose reabsorption, or any combination of the foregoing (such as those described in U.S. Patent Publication No. 2005/0143424). Suitable second hypoglycemic agents include insulin secretion-promoting agents, insulin resistance-ameliorating agents, insulin mimetics, α-glucosidase inhibitors, glucogenesis inhibitors, and any combination of any of the foregoing. According to one embodiment, the solid dosage form includes a sulfonyl urea, meglitinide analogue, biguanide (preferably metformin), or any combination of any of the foregoing. According to a preferred embodiment, the solid dosage form includes metformin.

Also provided is a pharmaceutical formulation, such as a solid dosage unit form, comprising the microparticles or nanoparticles of the present invention and/or having the disintegration times discussed above. The dosage unit form may be in the form of a tablet, capsule, powder, or sachet. The dosage unit form may have, alone or in combination, one or more enteric coatings, disintegrants, super disintegrants (such as sodium starch glycolate or croscarmellose sodium), and extra particle super disintegrants.

In one embodiment, the solid oral dosage unit form is a fast disintegrating tablet. In another embodiment, the solid dosage unit form has a controlled or delayed release.

According to one embodiment, the present invention provides a tablet comprising the aforementioned particles and a disintegrant. In one embodiment, the disintegrant is a super disintegrant, such as sodium starch glycolate (Primojel® available from Azebe UK Ltd. of South Humberside, UK), croscarmellose sodium (Primellose® available from Azebe UK Ltd. of South Humberside, UK), or an extra particle super disintegrant.

Another embodiment is a solid dosage form comprising a therapeutically effective amount of insulin and a delivery agent compound, where the solid dosage form has a disintegration time of at least 60 seconds when administered orally. The solid dosage form may have an enteric coating or be a surface eroding formulation. The solid dosage form may further comprise one or more enzyme inhibiting agents.

Yet another embodiment is a solid dosage form comprising a therapeutically effective amount of insulin and a delivery agent compound, where the solid dosage form does not substantially disintegrate or dissolve in the stomach but does disintegrate or dissolve in the small intestine. The solid dosage form may have an enteric coating or be a surface eroding formulation. The solid dosage form may further comprise one or more enzyme inhibiting agents.
The present specification further discloses a method for administering an active agent to an animal, particularly an animal in need of the active agent, by administering a pharmaceutical formulation comprising the microparticles or nanoparticles of the present invention and/or those having the disintegration times discussed above (i.e. those having a controlled or sustained release). Oral administration is a preferred route of administration.
The present specification further discloses a method of treating a disease or for achieving a desired physiological effect in an animal by administering a pharmaceutical formulation of the present invention, including solid unit dosage forms comprising the microparticles or nanoparticles of the present invention and/or those having the disintegration times discussed above (i.e. those having a controlled or sustained release). The specification further discloses a method of increasing the oral bioavailability of active agents by orally administering a pharmaceutical formulation of the present invention.
The present specification further discloses a method of treating diabetes and/or reducing the incidence of systemic hyperinsulinemia associated with chronic dosing of insulin in a mammal (such as in a human, particularly a human in need thereof) by administering to the mammal a therapeutic effective amount of an insulin-containing pharmaceutical formulation of the present invention, e.g., those comprising the microparticles or nanoparticles of the present invention and/or those having the disintegration times discussed above. In one embodiment, the delivery agent compound is the free acid of 4-CNAB or a pharmaceutically acceptable salt thereof. The pharmaceutical formulation may be administered on a chronic basis.
The present specification further discloses a method of treating impaired glucose tolerance, early stage diabetes, or late stage diabetes or achieving glucose homeostasis in a mammal (such as in a human, particularly in need thereof) by administering to the mammal a therapeutic effective amount of an insulin-containing pharmaceutical formulation of the present invention, such as a pharmaceutical formulation comprising the microparticles or nanoparticles of the present invention and/or having the disintegration times discussed above. In one embodiment, the delivery agent compound is the free acid of 4-CNAB or a pharmaceutically acceptable salt thereof. The pharmaceutical formulation may be administered on a chronic basis.
The present specification further discloses a method of treating a human diabetic patient by orally administering to the human diabetic patient on a chronic basis a therapeutic effective amount of an insulin-containing pharmaceutical formulation described herein.
The present specification further discloses a method of preparing the micro- and nano particles of the present invention by drying a solution of a delivery agent compound and an active agent, for example, until a solid is formed, and optionally, isolating the particles. Preferably, the mixture is homogenous (e.g., the delivery agent compound and the active agent are uniformly distributed throughout the mixture). The method includes co-drying a mixture of the delivery agent compound, the active agent, and a solvent. Suitable solvents include hydroxylic solvents, water, and mixtures thereof.
According to one embodiment, the mixture is dried at from about 10 to about 40° C (e.g., at room temperature). Preferably, the drying is performed at a controlled temperature. According to one embodiment, the drying is performed over an inert gas (preferably nitrogen gas). The dried material may optionally be milled and/or sieved to obtain the desired particle size. This method results in particles containing a homogeneous mixture of the delivery agent compound and the active agent.

Another method of preparing the micro- and nano-particles of the present invention is by lyophilizing a mixture of the delivery agent compound, the active agent, and a solvent. Suitable solvents include hydroxylic solvents, water, and mixtures thereof.

Yet another method of preparing the micro- and nano-particles of the present invention is by (1) dissolving a delivery agent compound and an active agent in a supercritical fluid, and (2) decreasing the system pressure to deposit the delivery agent compound and active agent as extremely fine particles. The deposition is a result of the rapid expansion of the supercritical solution.

The following embodiments are collectively referred to herein as the "solid pharmaceutical composition embodiments".

The above features and many other attendant advantages of the invention will become better understood by reference to the following detailed description when taken in conjunction with the accompanying drawings.

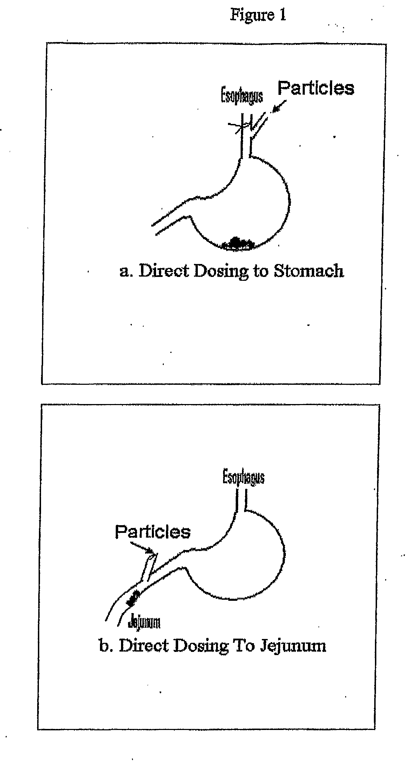

Figure 1 depicts a schematic of direct dosing to the stomach and the jejunum.

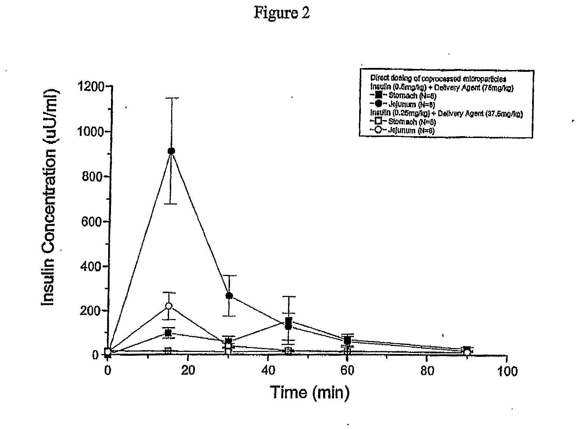

Figure 2 is a graph of the concentration of insulin level (±SEM) following direct dosing of coprocessed microparticles to the stomach and the jejunum over time.

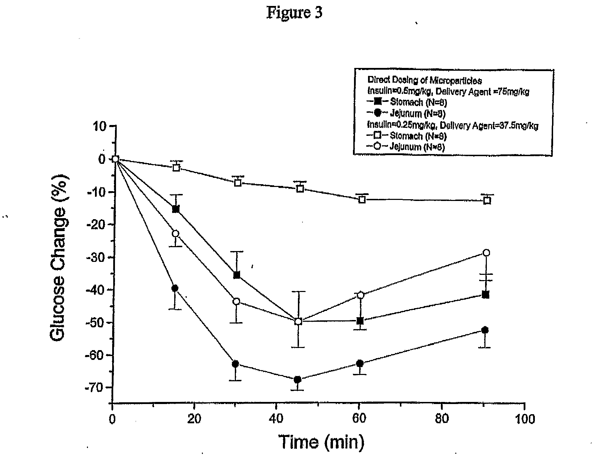

Figure 3 is a graph of the change in glucose level (±SEM) following direct dosing of coprocessed microparticles to the stomach and the jejunum over time.

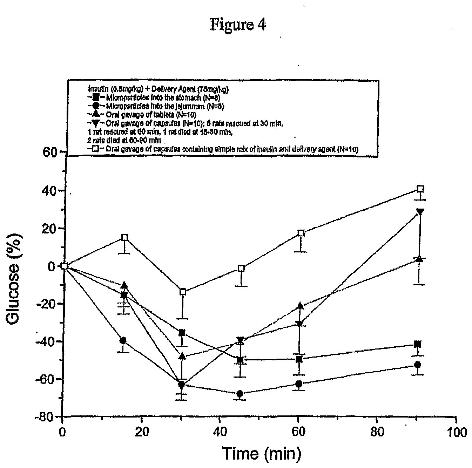

Figure 4 is a graph of the change in glucose (±SEM) following oral gavage from 3 different dosage forms: 1) a tablet made by compressing insulin and carrier, 2) a capsule containing microparticles of coprocessed insulin and carrier, and 3) a capsule containing a simple mixture of insulin and carrier, over time.

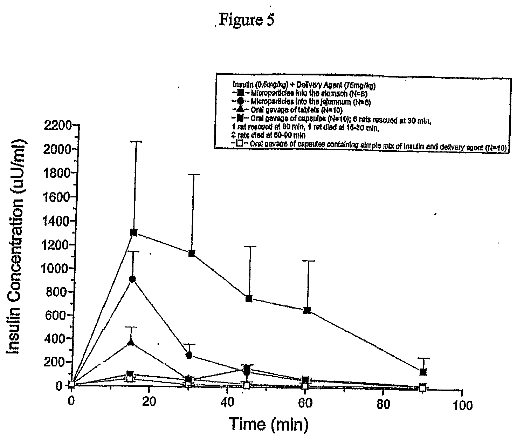

Figure 5 is a graph of the insulin level (±SEM) following oral gavage from 3 different dosage forms: 1) a tablet made by compressing insulin and carrier, 2) a capsule containing microparticles of coprocessed insulin and carrier, and 3) a capsule containing a simple mixture of insulin and carrier, over time.

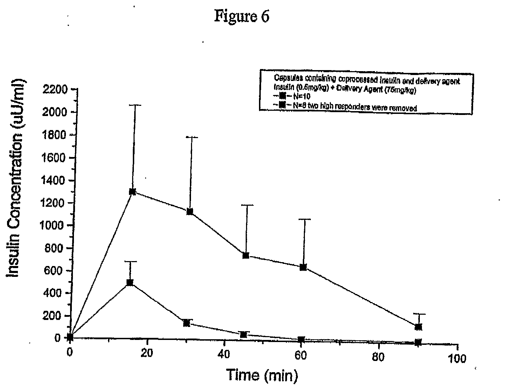

Figure 6 is a graph of the insulin level (±SEM) following oral gavage of a capsule containing microparticles of coprocessed insulin and carrier over time. Two of the ten rats exhibited significantly high insulin absorption. The average values with (N=10) and without (N = 8) inclusion of these two high responders are depicted in the graph.

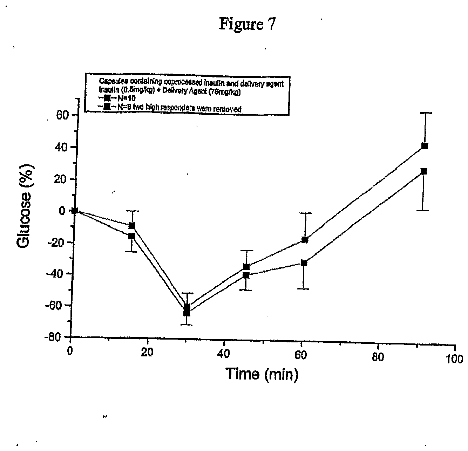

Figure 7 is a graph of the change in glucose (±SEM) following oral gavage of a capsule containing microparticles of coprocessed insulin and carrier over time. Two of the ten rats exhibited significantly high insulin absorption. The average values with (N = 10) and without (N = 8) inclusion of these two high responders are depicted in the graph.

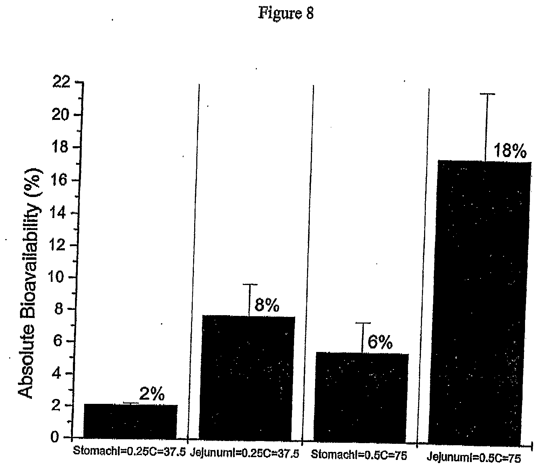

Figure 8 is a chart of the estimated absolute bioavailability (±SEM) from in situ dosing of coprocessed insulin and carrier to the stomach and the jejunum. Two compositions were evaluated: 1) insulin (0.25mg/kg) + delivery agent (37.5mg/kg), and 2) insulin (0.5mg/kg) + delivery agent (75mg/kg).

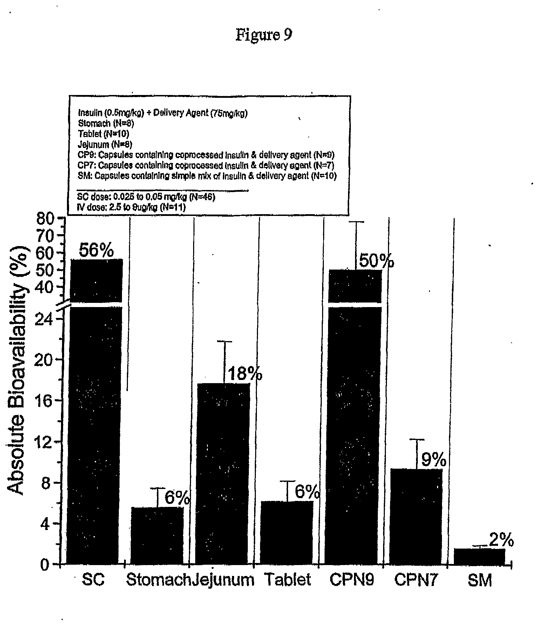

Figure 9 is a chart of the estimated absolute bioavailability of insulin level (±SEM) from 1) subcutaneous administration, 2) direct dosing to the stomach, 3) direct dosing to the jejunum, 4) a tablet made by compressing insulin and carrier, 5) a capsule containing microparticles of coprocessed insulin and carrier with and without inclusion of the two high responders, and 6) a capsule containing a simple mixture of insulin and carrier.

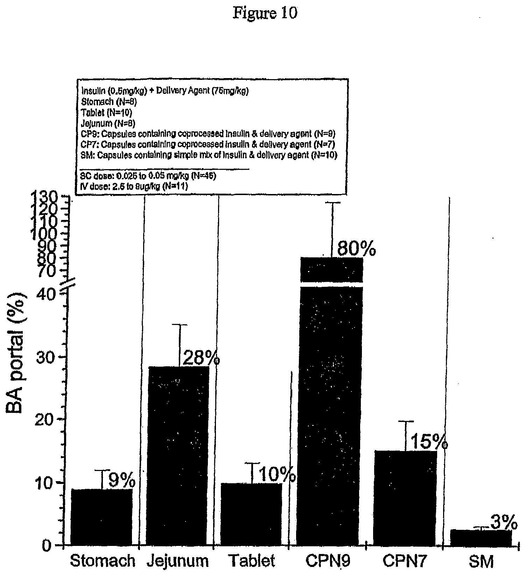

Figure 10 is a chart of the estimated bioavailability of insulin in the portal vein (±SEM) from 1) direct dosing to the stomach, 2) direct dosing to the jejunum, 3) a tablet made by compressing insulin and carrier, 4) a capsule containing microparticles of coprocessed insulin and carrier with and without inclusion of the two high responders, and 5) a capsule containing a simple mixture of insulin and carrier.

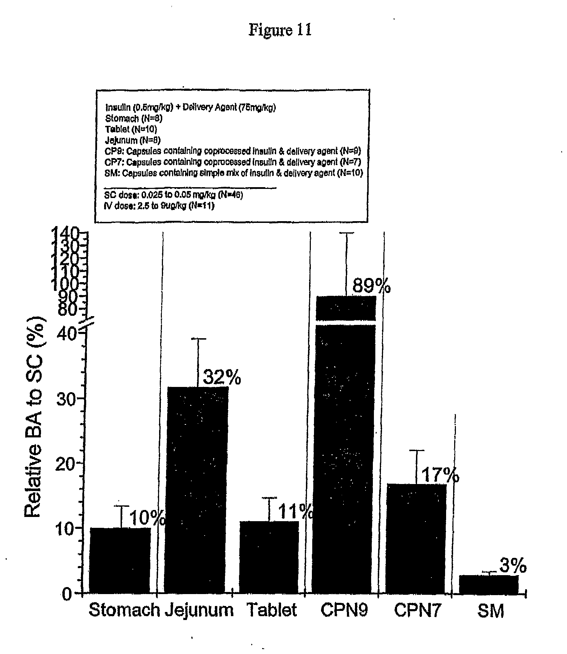

Figure 11 is a chart of the estimated bioavailability (±SEM) of insulin relative to subcutaneous administration from 1) direct dosing to the stomach, 2) direct dosing to the jejunum, 3) a tablet made by compressing insulin and carrier, 4) a capsule containing microparticles of coprocessed insulin and carrier with and without inclusion of the two high responders, and 5) a capsule containing a simple mixture of insulin and carrier.

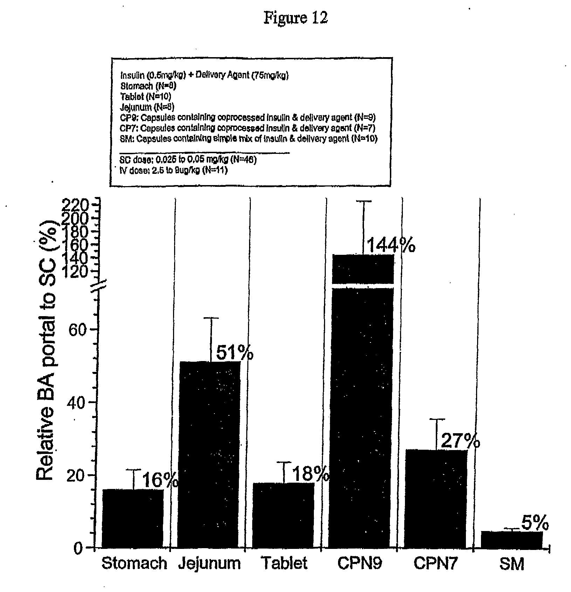

Figure 12 is a chart of the estimated bioavailability of insulin in the portal vein relative to subcutaneous administration (±SEM) from 1) direct dosing to the stomach, 2) direct dosing to the jejunum, 3) a tablet made by compressing insulin and carrier, 4) a capsule containing microparticles of coprocessed insulin and carrier with and without inclusion of the two high responders, and 5) a capsule containing a simple mixture of insulin and carrier.

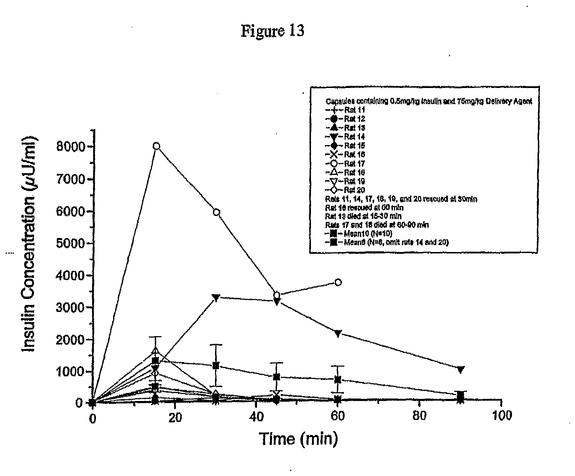

Figure 13 is a graph of the individual insulin levels following oral gavage of a capsule containing microparticles of coprocessed insulin and carrier over time. Rat 14 and rat 17 exhibited significantly high insulin absorption. The average values with (N = 10) and without (N = 8) inclusion of these two high responders are depicted in the graph.

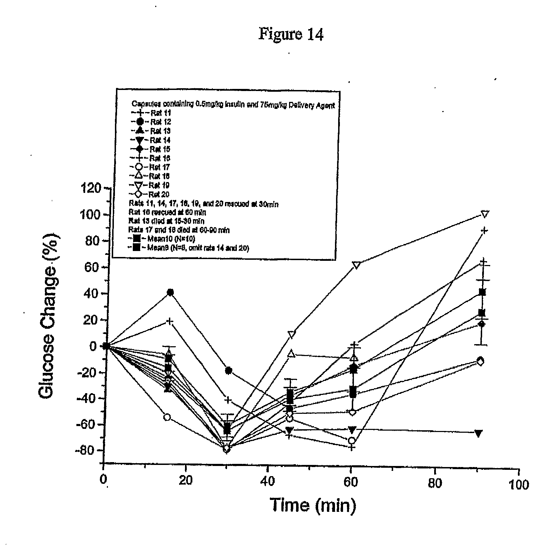

Figure 14 is a graph of the individual glucose change following oral gavage of a capsule containing microparticles of coprocessed insulin and carrier over time. Rat 14 and rat 17 exhibited significantly high insulin absorption. The average values with (N= 10) and without (N = 8) inclusion of these two high responders are depicted in the graph.

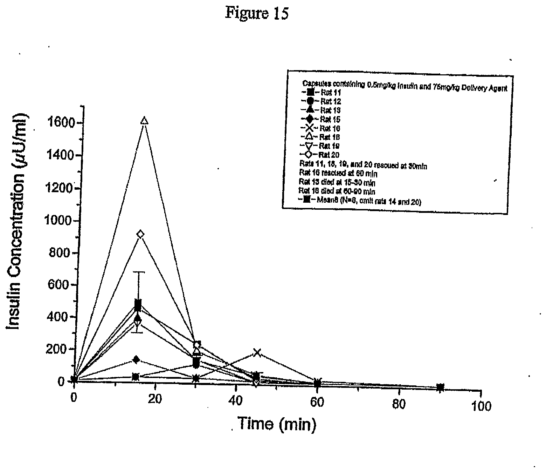

Figure 15 is a graph of the individual insulin level following oral gavage of a capsule containing microparticles of coprocessed insulin and carrier over time. Rats 14 and 17 were omitted. The average value from N = 8 is depicted in the graph.

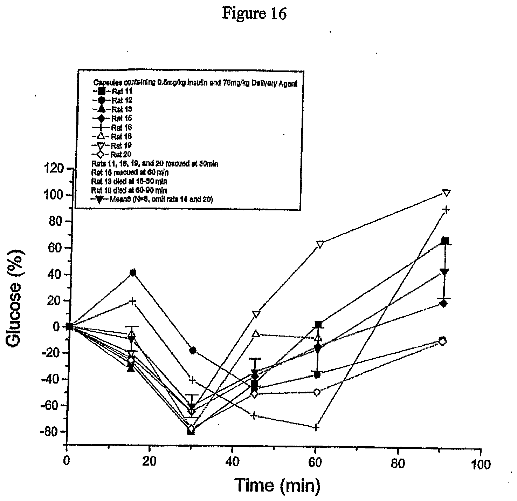

Figure 16 is a graph of the individual glucose change following oral gavage of a capsule containing microparticles of coprocessed insulin and carrier over time. Rats 14 and 17 were omitted. The average value from N=8 is depicted in the graph.

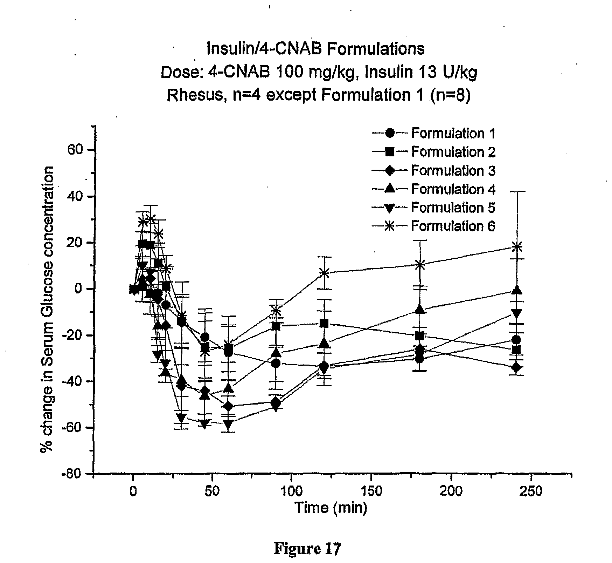

Figure 17 is a graph depicting the changes over time in serum glucose levels in rhesus monkeys that have been fed formulations 1-6, described below, containing insulin and a delivery agent. These formulations have varying disintegration times.

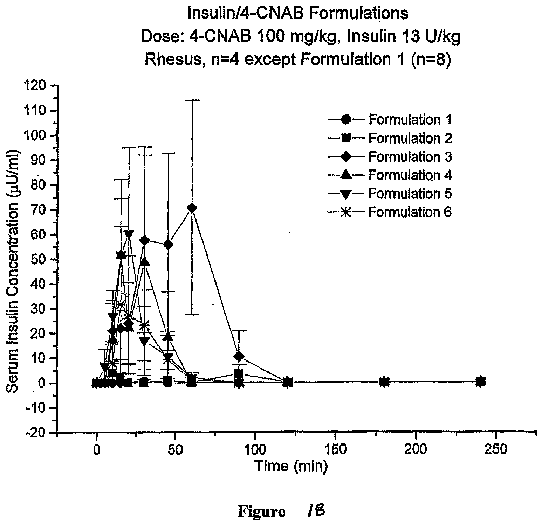

Figure 18 is a graph depicting the changes over time in serum insulin concentration rhesus monkeys that have been fed formulations 1-6, described below, containing insulin and a delivery agent. These formulations have varying disintegration times.

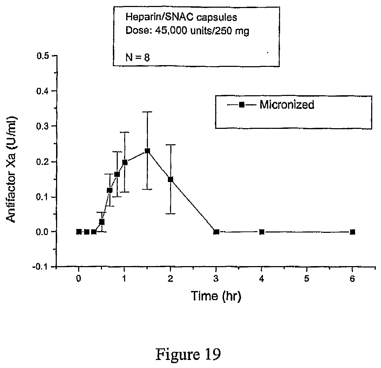

Figure 19 is a graph of anti-factor Xa activity (U/ml) versus time in monkeys after administration of the SNAD/heparin formulation described in Example 10.

### Definitions

The "particles," "micro-beads," and "granules" described herein may be any shape and can include one or more ingredients in addition to the delivery agent compound and/or active agent. The specific ingredients of any given particle, micro-bead, or granule, may also depend on the processes used and will not necessarily be the same in each individual particle, micro-bead, or granule from a batch.

For example, where particles, micro-beads, or granules of an active agent are prepared separately from particles, micro-beads, or granules of a delivery agent compound, the active agent particles, micro-beads, or granules will, generally, not comprise delivery agent compound, and the delivery agent particles, micro-beads, or granules will, generally, not comprise active agent, though each particle, micro-bead, or granule may comprise other ingredients, as disclosed herein.

In other embodiments, particles, micro-beads, or granules may be formed from a solution, suspension or mixture, in liquid or dry form which comprises at least an active agent and a delivery agent compound. Thus, for example, any given particle, micro-bead, or granule comprises both active agent and delivery agent compound, and may further comprise one or more other ingredients.

The terms "diameter" and "median particle size" are generally used to refer to the dimensions of particles, micro-beads, and granules. The "median particle size" or "diameter" was determined as follows for Examples 8, 9, 10.
Instrument: Mastersizer 2000 (EQ 202, model MS2K, serial number 34315-67).
Manufacturer: MALVERN instruments, England
Software: Mastersizer 2000
Accessory: Scirocco 2000 (A) (model ADA 2000, serial number 34270/73)
Dispersant: Dry dispersion
Analysis model: General purpose
Particle RI: 1.520
Obscuration: 1 - 6%
Standards: Malvern Quality Audit Standard for Sample Dispersion Units

The Malvern Mastersizer 2000 determines particle size by laser diffraction and model fitting. A well-dispersed sample in any two-phase system (e.g., powders, suspensions, or emulsions) is introduced into the path of a *He-Ne* laser focused with a lens of a length suitable for particle sizes present in the sample. The scattering pattern of particles in the laser path is measured by an array of detectors, with each detector measuring data from a particular range of angles.

The Malvern apparatus assumes that the particles being measured are perfect spheres. For non-spherical particles the resulting particle size distribution may be different from those obtained by methods based on others principles. The electronic measurements will often have to be accompanied by microscopic investigation to determine the type of particles being investigated. For irregularly shaped particles, the particle size data obtained from Mastersizer 2000 will be interpreted as the diameter of an imaginary sphere that is equivalent in volume to the measured particle. (Note: d(0.1) is the size of particle for which 10% of the sample is below this size, d(0.5) is the size of particle for which 50% of the sample is below this size, and d(0.9) is the size of particle for which 90% of the sample is below this size.

Generally, this apparatus measures one dimension of a, e.g., particle as it travels past a laser; i.e., it measures the length of a straight line through the particle. For irregular particles, this results in a variation of results since the orientation of a particle relative to the laser may result in the single measurement being taken of that individual particle's longest, shortest, or any other dimension. However, a measurement is taken of a number of particles and a median diameter or size is calculated. Thus, "size" or "diameter" figures are estimates of the median "size" or "diameter" of particles. Alternatively, "diameter" or "size" was measured by a sieve method described in Example 1. "Diameter" should not be read to necessarily imply a spherical shape or a circular dimension, though in certain embodiments, e.g., particles may have rounded edges or generally spherical shapes.

It should be understood, also, that the invention is not limited to particles, micro-beads, or granules which fall within a narrow range of "sizes" or "diameters". Thus, for example, some embodiments may comprise, depending at least on the ingredients and processes used, some particles which fall within, for example, both the nanometer and micrometer scale, in the same batch. The actual "sizes" or "diameters" of the individual particles may fall within a relatively narrow or relatively large range.

As used herein and in the appended claims, the singular forms "a," "an," and "the," include plural referents unless the context clearly indicates otherwise. Thus, for example, reference to "a particle" includes one or more of such particles, reference to "an" active agent includes one or more of such active agents, and "a" delivery agent includes one or more delivery agents,

The term "about" generally means within 10%, preferably within 5%, and more preferably within 1% of a given value or range.

The term "hydrate" as used herein includes (i) a substance containing water combined in the molecular form and (ii) a crystalline substance containing one or more molecules of water of crystallization or a crystalline material containing free water.

The term "solvate" as used herein includes a molecular or ionic complex of molecules or ions of a solvent with molecules or ions of the delivery agent compound or salt thereof, or hydrate or solvate thereof.

The term "delivery agent" refers to any of the delivery agent compounds disclosed herein.

The term "SNAC" refers to the monosodium salt of N-(8-[2-hydroxybenzoyl)-amino)caprylic acid, including the various polymorphic forms of the monosodium salt described in U.S. Provisional Application No. 60/569,476, filed May 6, 2004 unless otherwise indicated.

The term "SNAD" refers to the monosodium salt of N-(10-[2-hydroxybenzoyl]-amino)decanoic acid, unless otherwise indicated. The term "disodium salt of SNAD" refers to the disodium salt of N-(10-[2-hydroxybenzoyl]-amino)decanoic acid.

The term "5-CNAC" refers to the monosodium salt of N-(8-[2-hydroxy-5-chlorobenzoyl]-amino)octanoic acid, unless otherwise indicated.

The term "4-CNAB" refers to the monosodium salt of sodium N-4-[(2-hydroxy-4-chlorobenzoyl)amino]butanoate, including anhydrous, monohydrate, and isopropanol solvates thereof and various polymorphic forms of the monosodium salt described in International Publication No. WO 03/057650, unless otherwise indicated.

An "effective amount of active agent" is an amount of active agent which is effective to treat or prevent a condition in a living organism to whom it is administered over some period of time, e.g., provides a therapeutic effect during a desired dosing interval.

The term "insulin" refers to all forms of insulin, including naturally derived insulin and synthetic forms of insulin, such as those described in U.S. Patent Nos. 4,421,685, 5,474,978, and 5,534,488.

The term "insulin derivatives" refers to insulin-derived proteins and peptides with insulin actions, and include, for example, lispro, B10Asp and HOE-901.

An "effective amount of delivery agent" is an amount of the delivery agent which enables and/or facilitates the absorption of a desired amount of active agent via any route of administration (such as those discussed in this application including the oral (e.g., across a biological membrane in the gastrointestinal tract), nasal, pulmonary, dermal, buccal, vaginal, and/or ocular route).

The terms "alkyl" and "alkenyl" as used herein include linear and branched alkyl and alkenyl substituents, respectively.

The phrase "pharmaceutically acceptable" refers to additives or compositions that are physiologically tolerable when administered to a mammal.

The phrase "substantially disintegrate" means that about 75% to about 95% of the total volume of the tablet will break apart and dissolve into its component parts (e.g. insoluble coated particles, insoluble disintegrant, etc.), and the tablet is no longer intact except for small aggregates.

"Surface eroding formulation" refers to formulations that do not disintegrate but instead erode, e.g., the formulation dissolves from the surface over a pre-determined period of time and the tablet generally remains intact and retains its overall shape. The surface eroding formulations allow for sustained release of an active agent over the pre-determined time period.

The terms "micronize" and "micronized" generally refer to a process, or particles which have been processed, such that their diameters/sizes are within the general range of microparticles and/or nanoparticles.

The term "microparticle" generally includes particles having a diameter ranging from about 1 to about 999 micrometers (microns, µm).

The term "nanoparticle" generally includes particles having a diameter ranging from about 1 to about 999 nanometers (nm)..

The term "insulin derivatives" includes insulin-derived proteins and peptides with insulin actions, and include, for example, lispro, B10Asp and HOE-901.

"Insulin secretion-promoting agents" exert their hypoglycemic action, by mainly influencing pancreatic β-cells to promote insulin secretion into blood, and include, for example, sulfonylureas (for example, tolbutamide, chlorpropamide, glibenclamide (glyburide), glipizide, glimeperide, and gliclazide); and meglitinide analogues (for example, repaglinide, nateglinide, meglitinide and mitiglinide (KAD-1229))). Other insulin secretion-promoting agents are, for example, K⁺-ATP channel inhibitors (for example, BTS-67-582), glucagon-like peptide-1 receptor agonists (for example, glucagon-like peptide-1, exendin-4 and NN-2211) and dipeptidyl peptidase-IV inhibitors with an effect of enhancing the action of glucagon-like peptide-1. According one embodiment, the insulin secretion-promoting agent is a sulfonylurea or meglitinide analogue.

The term "insulin resistance-ameliorating agents" includes agents exerting hypoglycemic action by enhancing the action of insulin in target tissues, and include for example peroxisome proliferator activator receptor (PPAR)-γ agonists (for example, thiazolidine-based compounds such as pioglitazone, rosiglitazone, and ciglitazone; or non-thiazolidine-based compounds such as GI-262570, JTT-501, YM-440, NN-622 and KRP-297), PPAR-γ antagonists and protein tyrosine phosphatase inhibitors. The insulin resistance-ameliorating agents include, for example, pharmaceutical agents with a function ameliorating insulin resistance, for example biguanides (for example, metformin, phenformin and buformin, preferably metformin), PPAR-α agonists (fibrate-series compounds such as simfibrate, clofibrate, bezafibrate and clinofibrate and non-fibrate-series compounds), anti-obesity agents (for example, 5-hydroxytryptamine (5-HT) reuptake inhibitors such as sibutramine, lipase inhibitors such as orlistat and adrenalin β-receptor agonists such as AJ-9677). Preferred insulin resistance-ameliorating agents include, biguanides, such as metformin.

The term "insulin mimetics" refers to agents expressing the hypoglycemic action through physiological insulin action, namely the action promoting glucose uptake into cells, in a manner more or less independent to insulin, except for insulin derivatives, and include for example insulin receptor-activating agents (for example, CLX-0901 and L-783281) and vanadium.

The term "α-glucosidase inhibitors" refers to agents expressing the hypoglycemic action through suppression of glucose absorption into bodies, mainly via the inhibition of α-glucosidase in the intestinal tube and include, for example, acarbose, voglibose and miglitol.

The term "glucogenesis inhibitors" refers to agents expressing hypoglycemic action mainly through the inhibition of glucogenesis, and include for example glucagon secretion suppressors (for example, M&B-39890A and octreotide), fatty acid decomposition inhibitors (for example, nicotinic acid derivatives and carnitine palmitoyltransferase-1 inhibitor) and glucose-6-phosphatase inhibitors.

The term "inhibitor of renal glucose reabsorption" refers to agents which inhibit glucose reabsorption in uriniferous tubules. The primary action of the inhibitor of renal glucose reabsorption is not involved in the promotion of the uptake into target tissue cells, the suppression of the absorption from intestinal tube, or the hypoglycemic action via the suppression of the synthesis in tissues. Suitable inhibitors of renal glucose reabsorption include, those described in U.S. Patent Publication No. 2005/0143424.

### Delivery Agent Compounds

Non-limiting examples of delivery agent compounds include N-(8-[2-hydroxybenzoyl]amino)caprylic acid, N-(10-[2-hydroxybenzoyl]amino)decanoic acid, 8-(2-hydroxy-4-mcthoxybenzoylamino)octanoic acid, 8-(2-hydroxy-5-chlorobenzoyl-amino)octanoic acid, 4-[(2-hydroxy-4-chlorobenzoyl)amino]butanoic acid, and salts thereof. Preferred salts include monosodium and disodium salts.

According to one embodiment, the delivery agent compound is N-(8-[2-hydroxybenzoyl]amino)caprylic acid or a pharmaceutically acceptable salt thereof.

According to another embodiment, the delivery agent compound is N-(10-[2-hydroxybenzoyl]amino)decanoic acid or a pharmaceutically acceptable salt thereof.

According to another embodiment, the delivery agent compound is 4-[(2-hydroxy-4-chlorobenzoyl)amino]butanoic acid or a pharmaceutically acceptable salt thereof.

According to another embodiment, the delivery agent compound is 8-(2-hydroxy-5-chlorobenzoylamino)octanoic acid or a pharmaceutically acceptable salt thereof.

The delivery agent compounds may be in the form of the carboxylic acid or pharmaceutically acceptable salts thereof, such as sodium salts, and hydrates and solvates thereof. The salts may be mono- or multi-valent salts, such as monosodium salts and disodium salts (e.g., the disodium salt of 8-(2-hydroxy-5-chlorobenzoylamino)-octanoic acid, the disodium salt of N-(8-[2-hydroxybenzoyl]amino)caprylic acid, the disodium salt of N-(10-[2-hydroxybenzoyl]amino)decanoic acid). See, for example, International Publication No. WO 00/59863. The delivery agent compounds may contain different counter ions chosen for example due to their effect on modifying the dissolution profile of the carrier.

The delivery agent compounds may be prepared by methods known in the art, such as those discussed in the aforementioned publications (e.g., International Publication Nos. WO 98/34632, WO 00/07979, WO 01/44199, WO 01/32596, WO 02/02509, WO 02/20466, and WO 03/045306). SNAC, SNAD, 4-CNAB, and the free acid and other salts thereof may be prepared by methods known in the art, such as those described in U.S. Patent Nos. 5,650,386 and 5,866,536 and International Publication No. WO 02/02509.

Salts of the delivery agent compounds of the present invention may be prepared by methods known in the art. For example, sodium salts may be prepared by dissolving the delivery agent compound in ethanol and adding aqueous sodium hydroxide.

The delivery agent compound may be purified by recrystallization or by fractionation on one or more solid chromatographic supports, alone or linked in tandem. Suitable recrystallization solvent systems include acetonitrile, methanol, and tetrahydrofuran. Fractionation may be performed on a suitable chromatographic support such as alumina, using methanol/n-propanol mixtures as the mobile phase; reverse phase chromatography using trifluoroacetic acid/acetonitrile mixtures as the mobile phase; and ion exchange chromatography using water or an appropriate buffer as the mobile phase. When anion exchange chromatography is performed, preferably a 0-500 mM sodium chloride gradient is employed.

### Active Agents

Active agents suitable for use in the present invention are biologically active agents, including pesticides, pharmacological agents, and therapeutic agents. Suitable active agents include those that are rendered less effective, ineffective or are destroyed in the gastro-intestinal tract by acid hydrolysis, enzymes and the like. Also included as suitable active agents are those macromolecular agents whose physiochemical characteristics, such as, size, structure or charge, prohibit or impede absorption when dosed orally.

For example, biologically active agents suitable for use in the present invention include proteins; polypeptides; peptides; hormones; polysaccharides, and particularly mixtures of muco-polysaccharides; carbohydrates; lipids; small polar organic molecules (i.e. polar organic molecules having a molecular weight of 500 daltons or less); other organic compounds; and particularly compounds which by themselves do not pass (or which pass only a fraction of the administered dose) through the gastro-intestinal mucosa and/or are susceptible to chemical cleavage by acids and enzymes in the gastro-intestinal tract; or any combination thereof.

Further examples include the following, including synthetic, natural or recombinant sources thereof: growth hormones, including human growth hormones (hGH), recombinant human growth hormones (rhGH), bovine growth hormones, and porcine growth hormones; growth hormone releasing hormones; growth hormone releasing factor, interferons, including α (e.g., interferon alfacon-1 (available as Infergen® from InterMune, Inc. of Brisbane, CA)), β and 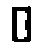 ; interleukin-1; interleukin-2; insulin, including porcine, bovine, human, and human recombinant, optionally having counter ions including zinc, sodium, calcium and ammonium; insulin-like growth factor, including IGF-1; heparin, including unfractionated heparin, heparinoids, dermatans, chondroitins, low molecular weight heparin, very low molecular weight heparin and ultra low molecular weight heparin; calcitonin, including salmon, eel, porcine and human; erythropoietin; atrial naturetic factor; antigens; monoclonal antibodies; somatostatin; protease inhibitors; adrenocorticotropin, gonadotropin releasing hormone; oxytocin; leutinizing-hormone-releasing-hormone; follicle stimulating hormone; glucocerebrosidase; thrombopoietin; filgrastim; prostaglandins; cyclosporin; vasopressin; cromolyn sodium (sodium or disodium chromoglycate); vancomycin; desferrioxamine (DFO); bisphosphonates, including alendronate, tiludronate, etidronate, clodronate, pamidronate, olpadronate, and incadronate; parathyroid hormone (PTH), including its fragments; anti-migraine agents such as BIBN-4096BS and other calcitonin gene-related proteins antagonists; glucagon-like peptide 1 (GLP-1); antimicrobials, including antibiotics, anti-bacterials and anti-fungal agents; vitamins; analogs, fragments, mimetics or polyethylene glycol (PEG)-modified derivatives of these compounds; or any combination thereof. Non-limiting examples of antibiotics include gram-positive acting, bacteriocidal, lipopeptidal and cyclic peptidal antibiotics, such as daptomycin and analogs thereof.

According to one embodiment, the active agent is insulin.

According to another embodiment, the active agent is heparin, such as unfractionated heparin or low molecular weight heparin.

The amount of active agent used in a pharmaceutical composition or dosage unit form of the present invention is an amount effective to treat the target indication. However, the amount can be less than that amount when the composition is used in a dosage unit form because the dosage unit form may contain a plurality of delivery agent compound/ active agent, such compositions may contain a divided effective amount. The total effective amount can then be administered in cumulative units containing, in total, an effective amount of active agent. Moreover, those skilled in the field will recognize that an effective amount of active agent will vary with many factors including the age and weight of the animal, the animal's physical condition, as well as other factors.

The total amount of active agent to be used of can be determined by methods known to those skilled in the art. However, because the compositions of the invention may deliver active agent more efficiently than compositions containing the active agent without the delivery agent, lower amounts of active agent than those used in prior dosage unit forms or delivery systems can be administered to the subject, while still achieving the same blood levels and/or therapeutic effects.

According to one embodiment, insulin is administered at a dose of about 0.025 to about 1.0 mg per kilogram of body weight of the recipient per day (mg/kg/day), about 0.06 to about 0.25 mg/kg/day, or about 0.09 to about 0.19 mg/kg/day (based on the weight of active agent). The desired dose may be administered either as a single or divided dose.

Generally an effective amount of delivery agent to facilitate the delivery of the active agent is administered with the active agent. According to one embodiment, the amount of delivery agent to active agent on a molar basis ranges from about 100:1 to about 1:1, from about 80:1 to about 2:1, or from about 20:1 to about 10:1. Delivery agent to active agent molar basis ranges may be higher than 100:1 for particular combinations of delivery agents and active agents. Alternatively, delivery agent to active agent ranges may be about 1:1 or lower, such as, e.g., 0.1:1 or lower, with particular combinations of delivery agents and active agents.

Dosage unit forms can also include any one or combination of excipients, disintegrants, lubricants, plasticizers, colorants, flavorants, taste-masking agents, sugars, sweeteners, and salts.

The compositions of the subject invention are useful for administering biologically active agents to any animals, including birds such as chickens, insects, fish, reptiles, mammals (including rodents, aquatic mammals, domestic animals such as dogs and cats, farm animals such as sheep, pigs, cows and horses, and preferably humans).

The present specification further discloses a method for the treatment or prevention of a disease or for achieving a desired physiological effect, such as those listed in the table 1 below, in an animal by administering the particles of the present invention. Preferably, an effective amount of the particles for the treatment or prevention of the desired disease or for achieving the desired physiological effect is administered. Specific indications for active agents can be found in the Physicians' Desk Reference (58^{th} Ed., 2004, Medical Economics Company, Inc., Montvale, NJ). The active agents in the table below include their analogs, fragments, mimetics, and polyethylene glycol-modified derivatives.

**Table 1**

| **Active Agent** | **Non-Limiting Examples of Disease And Physiological Effect** |
|---|---|
| Amylin and Amylin Agonists; | Obesity |
| Adrenocorticotropin; | High Cholesterol (To Lower Cholesterol) |
| Antigens; | Infection |
| Antimicrobials, including Antibiotics, Anti-Bacterials and Anti-Fungal Agents; non-limiting examples of Antibiotics include Gram-Positive Acting, Bacteriocidal, Lipopeptidal and Cyclic Peptidal Antibiotics, such as Daptomycin and Analogs thereof; | Infection Including Gram-Positive Bacterial Infection |
| Anti-Migraine Agents such as BIBN-4096BS and Other Calcitonin Gene-Related Proteins Antagonists, Sumatriptan Succinate; | Migraines |
| Antivirals including Acyclovir and Valacyclovir; | Viral Infections |
| Atrial Naturetic Factor; | Vasodilation |
| Argatroban; | Prophylaxis and treatment of thrombosis in patients with herapin-induced throbocytopenia ("HIT"), as well as an anticoagulant therapy in patients who have or are at risk for HIT undergoing percutaneous coronary intervention ("PCI"). Argatroban is also useful to treat thrombotic and isechemic stroke. |
| Bisphosphonates, including Alendronate, Clodronate, Etidronate, Ibandronate, Incadronate, Minodronate, Neridronate, Olpadronate, Pamidronate, Risedronate, Tiludronate, Zoledronate, EB1053, and YH529; | Osteoporosis; Paget's disease; Inhibits osteoclasts and Promotes osteoblastic activity; treat and/or prevent bone mineral density (bmd) loss; Breast cancer, including as adjuvant therapy for early stage breast cancer; Prostate cancer,; Testicular cancer; Colon cancer; Pancreatic cancer; Endometrial cancer; Small cell and non-small cell cancer of the lung; Ovarian cancer; Cervical cancer; Myeloid leukemia,; Lymphocyltic leukemia; Lymphoma; Hepatic tumors; Medullary thyroid carcinoma; Multiple myeloma; Melanoma retinoblastoma; Sarcomas of the soft tissue and bone; Hypercalcemia including hypercalcemia associated with malignancy; Osteolytic bone metastases and bone tumors; prevention of bone complications related to malignant osteolysis; Osteolytic lesions of multiple myeloma, fibrous dysplasia; pediatric osteogenesis imperfecta; hypercalcemia, urethral (urinary tract) malignancies, reflex sympathetic dystropy synodrome, acute back pain after vertebral crush fracture, chronic inflammatory joint disease, renal bone disease, extrosseous calcifications, analgesic, vitamin D intoxication, periarticular ossifications |
| BIBN4096BS -(1-Piperidinecarboxamide. N-[2-[ [5-amino-1-[[4-(4-pyridinyl)-1-piperazinyl)carbonyl]pentyl]amino]-1-[ (3,5-dibromo-4-hydroxyphenyl)methyl]-2-oxoethyl]-4(1,4-dihydro-2-oxo-3(2H0-quinazolinyl)-. [R-(R*,S*)]-); | Anti-migraine; calcitonin gene-related peptide antagonist |
| Calcitonin, including Salmon, Eel, Porcine and Human; | Osteoporosis; Diseases of the bone; bone pain; analgesic (including pain associated with osteoporosis or cancer) |
| Cholecystokinin (CCK) and CCK Agonists including CCK-8; | Obesity |
| Cromolyn Sodium (Sodium Or Disodium Chromoglycate); | Asthma; Allergies |
| CPHPC; | Reduction of amyloid deposits and systemic amyloidoisis often (but not always) in connection with Alzheimer's disease, Type II diabetes, and other amyloid-based diseases |
| Cyclosporine; | Transplant Rejection |
| Desferrioxamine (DFO); | Iron Overload |
| Dipeptidyl peptidase IV (DPP-4) inhibitors; | Diabetes; improving glycemic control (e.g. treating hypoglycemia), obesity |
| Erythropoietin; | Anemia |
| Exedin and Exedin Agonists, including Exendin-3 and Exendin-4; | Diabetes; Obesity |
| Filgrastim | Reduce infection in chemotherapy patients |
| Follicle Stimulating Hormone (recombinant and natural); | Regulate reproductive function |
| Gallium nitrate; | Osteoporosis; Paget's disease; Inhibits osteoclasts; Promotes osteoblastic activity, hypercalcemia, including cancer related hypercalcemia, urethral (urinary tract) malignancies; anti-tumors, cancers, including urethral and bladder cancers; lymphoma; malignancies (including bladder cancer); leukemia; management of bone metastases (and associated pain); muliple myeloma, attenuate immune response, including allogenic transplant rejections; disrupt iron metabolism; promote cell migration; wound repair; to attenuate or treat infectious processes of mycobacterium species, including mycobacterium tubercolosis, and mycobacterium avium complex |
| Glucagon; | Improving glycemic control (e.g. treating hypoglycemia and controlling hypoglycemic reactions); obesity; a diagnostic aid in the radiogical examination of the stomach, duodenum, small bowel and colon; treat acute poisoning with cardiovascular agents including, calcium channel blockers and beta blockers |
| Glucagon-Like Peptide 1 (GLP-1), Glucagon, and Glucagon-Like Peptide 2 (GLP-2); | Diabetes; Obesity |
| Glucocerebrosidase; | Gaucher disease (to metabolize lipoprotein) |
| Gonadotropin Releasing Hormone; | Ovulatory dysfunction (to stimulate ovulation) |
| Growth Hormone Releasing Factor; | Growth Disorders |
| Growth Hormone Releasing Hormones; | Growth Disorders |
| Growth hormones, including human growth hormones (hGH), recombinant human growth hormones (rhGH), bovine growth hormones, and porcine growth hormones; | Growth Disorders |
| Heparin, including unfractionated heparin, heparinoids, dermatans, chondroitins, low molecular weight heparin, very low molecular weight heparin ultra low molecular weight heparin and synthetic heparins including fondiparinux; | Thrombosis; prevention of blood coagulation |
| Insulin, including porcine, bovine, human, and human recombinant, optionally having counter ions including zinc, sodium, calcium and ammonium; | Diabetes; insulin resistance syndrome |
| Insulin-Like Growth Factor, including IGF-1; | Diabetes |
| Interferons, including α (e.g., interferon Alfacon-1 (available as Infergen® from Intermune, Inc. of Brisbane, Ca)), β, omega and 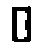 ; | Viral infection, including chronic cancer and multiple sclerosis |
| Interleukin-1; Interleukin-2; Interleukin-11; Interleukin-21; | Viral Infection; Cancer |
| Leutinizing Hormone and Leutinizing Hormone Releasing Hormone; | Regulate Reproductive Function |
| Leptin (OB Protein); | Obesity |
| Methyphenidate salt; | ADHD, Attention Deficit Disorder, Dementia, AIDS Dementia Complex, cognitive decline in HIV-AIDS |
| Monoclonal Antibodies including Retuxin, TNF-alpha soluble receptors; | To prevent graft rejection; cancer |
| Oxytocin; | Labor dysfunction (to stimulate contractions) |
| Parathyroid Hormone (PTH), including its fragments, including PTH 1-34 and PTH 1-38; | Osteoporosis; diseases of the bone |
| Peptide YY (PYY) including PYY Agonists and Fragment 3-36; | Obesity; Diabetes; Eating Disorders; Insulin Resistance Syndrome |
| Prostaglandins; | Hypertension |
| Protease Inhibitors; | Aids |
| Somatostatin; | Bleeding ulcer; erosive gastritis; variceal bleeding; diarrhea; acromegaly; TSH-secreting pituitary adenomas; secretory pancreatic tumors; carcinoid syndrome; reduce proptosis/ thyroid-associated ophthalmopathy; reduce macular edema/retinopathy |
| Thrombopoietin; | Thrombocytopenia |
| Vancomycin; | Treat or prevent antimicrobial-induced infections including methacillin-resistant *Staphalococcusaureus* and *Staph. epidermiditis* |
| Vasopressin; | Bed-wetting; antidiuretic |
| Vitamins; and | Vitamin deficiencies |
| Vaccines including those against Anthrax or *Y. Pestis*, Influenza, and Herpes. | Prevent and minimize disease |

### Controlled or Sustained Release Formulations

The solid dosage forms of the present invention may be formulated so as to prevent or retard break down in the stomach. Controlled release formulations suitable for use in the present invention may, for example, include an enteric coating or may be formulated to erode from the surface.

According to one embodiment, the solid oral dosage forms comprises a therapeutically effective amount of an active agent and a delivery agent, wherein the solid oral dosage form has a disintegration time of about 250 seconds to about 650 seconds when orally administered. In another embodiment, the disintegration time is about 350 to about 550 seconds when orally administered. In one embodiment the disintegration time is greater than 60 seconds when orally administered. In another embodiment, the disintegration time is greater than 400 seconds when orally administered. Disintegration time can be determined in water at 37 ± 2°C using the method described in USP <701>.

The solid dosage forms of the present invention may be covered by an enteric coating. The enteric coating may serve as the primary control for delaying the release of the drug composition or compositions in the solid dosage form. The enteric coating stays intact in the stomach and prevents or retards release into the stomach in the solid dosage form. Release of the active agent is delayed until the solid dosage form reaches the intestine. Once in the intestine, the higher pH causes release of the active agent. Enteric coatings include hydroxypropyl methylcellulose phthalate, hydroxypropyl methylcellulose acetate succinate, polyvinyl acetate phthalate, cellulose acetate trimellitate, cellulose acetate phthalate, poly(methacrylic acid-ethylacrylate), and poly(methacrylic acid-methyl methacrylate). Other enteric coatings which may be used in accordance with the present invention are described in U.S. Patent No. 5,851,579.

In one embodiment of the present invention, the enteric coating is applied to the entire tablet, or other dosage form. In one embodiment the enteric coating is applied to a multi-particulate system, such as a system comprising microparticles and/or nanoparticles discussed above.

The solid dosage forms of the present invention may be formulated to erode from the surface of the tablet (or other dosage uniform), or at the surface of the multi-particulate system (e.g. a system comprising microparticles discussed above). These surface erosion formulations slowly dissolve from the surface rather than disintegrate. By controlling the rate of surface erosion, release of the active agent and drug composition of the solid dosage form can be delayed. The surface erosion formulations can be formulated such that substantial release of the active agents or drug compositions do not occur until the solid oral dosage form reaches the intestines.

### Enzyme Inhibiting Agents

The solid dosage forms of the present invention (comprising the microparticles or nanoparticles of the present invention and/or having the disintegration times discussed above) may also include enzyme inhibiting agents. Enzyme inhibiting agents incorporated into the solid dosage unit forms may prevent the breakdown of insulin or other active agents that may be sensitive to enzymatic degradation. Enzyme inhibiting agents are described in U.S. Patent No. 6,458,383.

Generally, inhibitory agents can be divided into the following classes: inhibitors that are not based on amino acids, including P-aminobenzamidine, FK-448, camostat mesylate and sodium glycocholate; amino acids and modified amino acids, including aminoboronic acid derivatives and n-acetylcysteine; peptides and modified peptides, including bacitracin, phosphinic acid dipeptide derivatives, pepstatin, antipain, leupeptin, chymostatin, elastatin, bestatin, hosphoramindon, puromycin, cytochalasin potatocarboxy peptidase inhibitor, and amastatin; polypeptide protease inhibitors, including aprotinin (bovine pancreatic trypsin inhibitor), Bowman-Birk inhibitor and soybean trypsin inhibitor, chicken egg white trypsin inhibitor, chicken ovoinhibitor, and human pancreatic trypsin inhibitor; complexing agents, including EDTA, EGTA, 1,10-phenanthroline and hydroxychinoline; and mucoadhesive polymers and polymer-inhibitor conjugates, including polyacrylate derivatives, chitosan, cellulosics, chitosan-EDTA, chitosan-EDTA-antipain, polyacrylic acid-bacitracin, carboxymethyl cellulose-pepstatin, polyacrylic acid-Bowman-Birk inhibitor.

The choice and levels of the enzyme inhibitor are based on toxicity, specificity of the proteases and the potency of inhibition, and will be apparent to those skilled in the art.

Without wishing to be bound by theory, it is believed that an inhibitor can function solely or in combination as: a competitive inhibitor, by binding at the substrate binding site of the enzyme, thereby preventing the access to the substrate (examples of inhibitors believed to operate by this mechanism are antipain, elastatinal and the Bowman Birk inhibitor); a non-competitive inhibitor that can be simultaneously bound to the enzyme site along with the substrate, as their binding sites are not identical; and/or a complexing agent due to loss in enzymatic activity caused by deprivation of essential metal ions out of the enzyme structure.

### Examples

The following examples illustrate the invention. All parts are given by weight unless otherwise indicated.

### Example 1

### 1. Test Articles

### a. Co-processed insulin/delivery agent microparticles used for site specific, in situ experiment and oral gavage experiments

Recombinant human zinc insulin (50 mg) and sodium 4-CNAB (7.5 g) were dissolved in 50 ml of deionized water. The clear solution was dried with nitrogen flow at room temperature for 24 hours. The obtained coprocessed cake was milled into fine particles, which were then sieved through a 40/60 mesh screen to obtain microparticles of a specific size range. The size of the microparticles used in the current study ranged from 250 to 420 *µ*m. These microparticles contained by weight 0.55% of insulin, 9.5% of water and 89.5% of delivery agent. A total of approximately 90% (w/w) of insulin was recovered from this process.

Particles were measured by passing them through seives with different size openings (850 *µ*m, 425 *µ*m, 250 *µ*m, 150 *µ*m, 45 *µ*m). With this method, it can be determined that the median particle size ranges from about 45 to about 850 *µ*m, from about 45 to about 150 *µ*m, from about 150 to about 250 *µ*m, from about 250 to about 425 *µ*m, or from about 425 to about 850 *µ*m.

Insulin content in the microparticles was measured with reversed phase HPLC (Phenomenex column: Luna 3u C18 (2) 100Å, 150 x 4.6 mm, 3 micro; mobile phases: A, 0.1% TFA in water; B, 0.1% TFA in acetonitrile; Detector: UV280 nm). Water contents of the particles were measured with a 737 KF coulometer.

### b. Capsules loaded with the microparticles for oral gavage

Gelatin capsules (size #9) were used in the rat studies. The necessary amount of microparticles loaded manually into the gelatin capsules were determined based on an average rat body weight of 350 mg. Each loaded capsule contained approximately 16 mg of the microparticles (equivalent to 0.0875 mg of insulin).

### c. Insulin/Delivery Agent mini-tablets for oral gavage experiment

Insulin was well mixed with delivery agent at a ratio of 1:150 (w/w), which corresponded to 0.67% (w/w) of insulin. Based on an average rat body weight of 350 mg, a total amount of 26.43 mg of the mixed powder, which contained 0.175 mg of insulin and 26.26 mg of delivery agent, was directly compressed into tablets under a pressure of 6895 kPa (1000 psi) in a Carver press. The cylindrical mini-tablets were 2 mm in diameter and 6 mm in height.

### d. Capsules loaded with insulin/delivery agent physical blend for oral gavage experiment

Insulin was well mixed with delivery agent at a ratio of 1:150 (w/w). The amount of insulin and delivery agent mixture loaded manually into the gelatin capsules (size #9) were determined based on an average rat body weight of 350 mg. Each capsule contained 26.43 mg of the mixture (equivalent to 0.175 mg insulin).

### 2. Direct Dosing Procedures for In Situ Experiments

A schematic of the direct dosing procedure is shown in Figure 1. Surgery was carried out in a clean environment using a clean lab coat, mask, safety goggle, gloves and surgical cap. Anesthesia was induced to the Sprague Dawley rats with 5% isoflurane as an induction concentration, and maintained at 2% isoflurane in pure oxygen to the completion of the study.

### a. Stomach direct dosing

After the right jugular vein was catheterized for sampling blood, the skin over the esophagus and trachea was dissected, and the musculus digastricul venter rostralis (protective muscular bundles) was identified and dissected to make an access toward the esophagus. The esophagus was partially severed, and inserted with a 12 cm PE204 tubing for a segment of the esophagus measuring 6-9 cm. The dosing formulation was introduced through this tubing using a blunt wire to push in the microparticles. After dosing, the esophagus was ligated with a 3-0 silk suture for preventing any leakage from the stomach.

### b. Jejunum direct dosing

After the right jugular catheterization for the blood sampling, the abdominal cavity was opened by dissecting the linea alba toward the sternum, thus exposing the xiphoid cartilage. The most proximal segment of jejunum was first identified. A less vascularized section of the proximal jejunum was partially nipped, and a dosing tube was introduced toward the distal end. After dosing, the dosing tube was removed, and a 2 cm PE206 tubing was pushed in, and placed so that the nipped wound was located in the middle of both ends of the 2 cm tubing. A suture was tied around the tubing with jejunum at both ends, and the wound was closed with a drop of a vetbond™ tissue adhesive (available from 3M of St. Paul, Minnesota).

### 3. Oral Gavage Procedures

Studies were carried out in Sprague Dawley rats (body weight was approximately 350 grams) by oral gavage administration. The mini tablets or capsules were administrated orally in rats using a modified gavage tubing with a trocar. Rats were fasted for about 24 hours and anesthetized by intramuscular administration of ketamine (44 mg/kg) and thorazine (1.5 mg/kg). At pre-determined time intervals, blood samples were drawn from the tail artery and were appropriately prepared as either plasma or serum for glucose and insulin bioassays. The animal was sacrificed at the end of the experiment and rat gastrointestinal mucosa was observed for any sign of local toxicity.

### 4. Bioassay Procedures

Rat serum concentrations of insulin were determined using Insulin ELISA Test Kit (DSL Inc.). The limit of quantitation (LOQ) has been established at 12.5 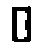 U/mL, with the calibrated linear range of the assay up to 250 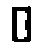 U/mL. Changes in blood glucose levels were measured using a glucometer.

### 5. Results

### a. Site Specific Study (In Situ) Results

The concentration of insulin and the change in glucose level following direct dosing of the coprocessed microparticles to the stomach and the jejunum are shown in Figures 2 and 3, respectively. The individual data are listed in Tables 2 to 5.

Insulin concentration from dosing to the jejunum reached a maximum value at the first sampling point (tₘₐₓ ≤ 15 min) from each formulation. The corresponding tₘᵢₙ of glucose occurred approximately 30 min. later.

**Table 2**

| Direct dosing of coprocessed microparticles to the stomach | | | | | | | | | | | | |
|---|---|---|---|---|---|---|---|---|---|---|---|---|
| *Insulin (0.5mg/kg), Delivery Agent (75mg/kg)) | | | | | | | | | | | | |
| 1) | Insulin | | | | | | | | | | | |
| | | | | | | | | | | | | |
| Insulin | | | | Stomach | | | | | | | | |
| Time (min) | Rat# 1 | #2 | #3 | #4 | #5 | #6 | #7 | #8 | mean | SD | SEM | CV |
| 0 | 12.5 | 12.5 | 12.5 | 12.5 | 12.5 | 12.5 | 12.5 | 12.9 | 0.0 | 0.0 | 0.0 | |
| 15 | 114.5 | 72.8 | 80.9 | 12.6 | 210.0 | 12.5 | 118.7 | 158.5 | 97.6 | 68.1 | 24.1 | 69.8% |
| 30 | 95.3 | 19.3 | 35.5 | 12.5 | 211.0 | 12.5 | 15.1 | 66.0 | 58.3 | 68.4 | 24.2 | 117.2% |
| 45 | 62.8 | 12.5 | 894.0 | 12.5 | 213.0 | 12.5 | 15.0 | 12.5 | 154.3 | 306.7 | 108.5 | 198.8% |
| 60 | 18.2 | 12.5 | 157.0 | 12.5 | 174.0 | 140.3 | 12.5 | 12.5 | 67.4 | 74.7 | 26.4 | 110.9% |
| 90 | 12.5 | 12.5 | 12.5 | 12.5 | 61.3 | 12.5 | 12.5 | 74.2 | 26.3 | 25.8 | 9.1 | 98.1% |
| AUC_{0→90} | 4780 | 2132 | 18970 | 1127 | 14438 | 4001 | 2795 | 5046 | 6661 | 6451 | 2281 | 96.8% |
| (two rat data were removed, rat #1-2 stomach) | | | | | | | | | | | | |
| | | | | | | | | | | | | |

| 2) | Glucose | | | | | | | | | | | |
|---|---|---|---|---|---|---|---|---|---|---|---|---|
| | | | | | | | | | | | | |
| Change from base line | | | | Stomach | | | | | | | | |
| Time (min) | Rat# 1 | #2 | #3 | #4 | #5 | #6 | #7 | #8 | Mean | SD | SEM | CV |
| 0 | 0 | 0 | 0 | 0 | 0 | 0 | 0 | 0 | 0 | 0 | 0 | |
| 15 | -9.9 | -21.0 | -12.6 | -38.1 | -6.6 | -24.8 | 3.0 | -13.9 | -15.5 | 12.5 | 4.4 | -80.6% |
| 30 | -44.3 | -43.8 | -25.8 | -56.1 | -9.8 | -54.4 | -3.4 | -47.6 | -35.7 | 20.2 | 7.1 | -56.6% |
| 45 | -75.0 | -50.9 | -30.2 | -73.6 | -17.1 | -73.6 | -14.8 | -64.2 | -49.9 | 25.8 | 9.1 | -51.6% |
| 60 | -80.7 | -51.3 | -31.1 | -66.1 | -18.7 | -72.8 | -21.9 | -55.1 | -49.7 | 23.5 | 8.3 | -47.3% |
| 90 | -62.3 | -32.1 | -35.2 | -59.7 | -20.3 | -63.6 | -29.5 | -28.3 | -41.4 | 17.5 | 6.1 | -42.3% |
| (two rat data were removed, rat #1-2 stomach) | | | | | | | | | | | | |

**Table 3**

| Direct dosing of coprocessed microparticles to the jejunum data | | | | | | | | | | | | |
|---|---|---|---|---|---|---|---|---|---|---|---|---|
| (Insulin (0.5mg/kg), Delivery Agent (75mg/kg)) | | | | | | | | | | | | |
| 1) | Insulin | | | | | | | | | | | |
| | | | | | | | | | | | | |
| Insulin | | | | | | Jejunum | | | | | | |
| Time (min) | #9 | #10 | #11 | #12 | #13 | #14 | #15 | #16 | mean | SD | SEM | CV |
| 0 | 12.5 | 12.5 | 12.5 | 12.5 | 12.5 | 12.5 | 12.5 | 12.5 | 0.0 | 0.0 | 0.0 | |
| 15 | 413.2 | 1193.3 | 669.4 | 1177.5 | 2270.6 | 228.9 | 954.4 | 374.9 | 910.3 | 661.1 | 233.8 | 72.6% |
| 30 | 354.3 | 148.4 | 70.5 | 64.7 | 481.0 | 168.4 | 782.9 | 57.4 | 265.9 | 258.2 | 91.3 | 97.1% |
| 45 | 79.5 | 28.0 | 20.5 | 26.5 | 170.8 | 148.6 | 531.6 | 12.5 | 127.3 | 174.3 | 61.6 | 137.0% |
| 60 | 23.1 | 14.7 | 12.5 | 16.8 | 71.6 | 117.0 | 200.1 | 12.5 | 58.5 | 68.4 | 24.2 | 116.9% |
| 90 | 12.5 | 12.5 | 12.5 | 12.5 | 30.8 | 12.5 | 37.5 | 12.5 | 17.9 | 10.2 | 3.6 | 56.8% |
| AUC_{0→90} | 13506 | 21158 | 11969 | 19690 | 46003 | 11102 | 39192 | 7235 | 21232 | 14054 | 4969 | 66.2% |
| (two rat data were removed, rat# 2 jejunum and rat# 4 jejunum) | | | | | | | | | | | | |
| | | | | | | | | | | | | |

| 2) | Glucose | | | | | | | | | | | |
|---|---|---|---|---|---|---|---|---|---|---|---|---|
| | | | | | | | | | | | | |
| Change from base line | | | | | | Jejunum | | | | | | |
| Time (min) | #9 | #10 | #11 | #12 | #13 | #14 | #15 | #16 | Mean | SD | SEM | CV |
| 0 | 0 | 0 | 0 | 0 | 0 | 0 | 0 | 0 | 0 | 0 | 0 | |
| 15 | -50.8 | -35.6 | -16.7 | -16.0 | -61.1 | -38.2 | -62.8 | -36.3 | -39.7 | 17.9 | 6.3 | -45.0% |
| 30 | -67.3 | -65.5 | -59.5 | -35.8 | -81.8 | -62.5 | -78.1 | -52.9 | -62.9 | 14.4 | 5.1 | -22.9% |
| 45 | -64.4 | -68.5 | -76.3 | -56.4 | -74.4 | -71.6 | -78.1 | -53.3 | -67.9 | 9.2 | 3.2 | -13.5% |
| 60 | -62.7 | -62.5 | -71.3 | -62.7 | -62.1 | -65.6 | -74.0 | -42.2 | -62.9 | 9.5 | 3.4 | -15.1% |
| 90 | -62.4 | -49.8 | -69.7 | -55.6 | -41.8 | -44.2 | -69.4 | -26.7 | -52.5 | 14.9 | 5.3 | -28.3% |
| (two rat data were removed, rat# 2 jejunum and rat# 4 jejunum) | | | | | | | | | | | | |

**Table 4**

| Direct dosing of coprocessed microparticles to the stomach | | | | | | | | | | | | |
|---|---|---|---|---|---|---|---|---|---|---|---|---|
| (Insulin (0.25mg/kg), Delivery Agent (37.5mg/kg)) | | | | | | | | | | | | |
| 1) | Insulin | | | | | | | | | | | |
| | | | | | | | | | | | | |
| stomach | | | | | | | | | | | | |
| Time (min) | sto-1 | sto-2 | sto-3 | sto-4 | sto-5 | sto-6 | sto-7 | sto-8 | mean | SD | SEM | CV |
| 0 | 12.5 | 12.5 | 41.2 | 21.5 | 12.5 | 12.5 | 12.5 | 12.5 | 17.2 | 9.5 | 3.4 | 55.4% |
| 15 | 12.5 | 20.8 | 14.7 | 12.5 | 12.5 | 12.5 | 26.4 | 12.5 | 16.0 | 4.9 | 1.7 | 30.7% |
| 30 | 12.5 | 12.5 | 12.5 | 12.5 | 12.5 | 12.5 | 12.5 | 12.5 | 12.5 | 0.0 | 0.0 | 0.0% |
| 45 | 48.8 | 12.5 | 12.5 | 12.5 | 12.5 | 12.5 | 12.5 | 12.5 | 17.0 | 12.0 | 4.3 | 70.4% |
| 60 | 12.5 | 12.5 | 12.5 | 12.5 | 12.5 | 12.5 | 12.5 | 12.5 | 12.5 | 0.0 | 0.0 | 0.0% |
| 90 | 12.5 | 12.5 | 12.5 | 12.5 | 12.5 | 12.5 | 12.5 | 12.5 | 12.5 | 0.0 | 0.0 | 0.0% |
| AUC_{0→90} | 1670 | 1250 | 1373 | 1193 | 1125 | 1125 | 1334 | 1125 | 1274 | 187 | 66.0 | 14.6& |
| | | | | | | | | | | | | |

| 2) | Glucose | | | | | | | | | | | |
|---|---|---|---|---|---|---|---|---|---|---|---|---|
| | | | | | | | | | | | | |
| Change from base line | | | | Stomach | | | | | | | | |
| Time (min) | Rat# 1 | #2 | #3 | #4 | #5 | #6 | #7 | #8 | mean | SD | SEM | CV |
| 0 | 0 | 0 | 0 | 0 | 0 | 0 | 0 | 0 | 0 | 0 | 0.0 | - |
| 15 | -2.2 | -12.3 | 2.2 | 0.8 | -2.2 | -12.3 | 2.2 | 0.8 | -2.9 | 5.7 | 2.0 | 196.5% |
| 30 | -14.4 | -10.1 | -6.5 | 0.8 | -14.4 | -10.1 | -6.5 | 0.8 | -7.6 | 5.6 | 1.9 | -73.7% |
| 45 | -15.8 | -8.8 | -12.7 | 0.0 | -15.8 | -8.8 | -12.7 | 0.0 | -9.3 | 5.9 | 2.1 | -63.4% |
| 60 | -15.8 | -11.4 | -17.9 | -5.8 | -15.8 | -11.4 | -17.9 | -5.8 | -12.7 | 4.6 | 1.6 | -36.2% |
| 90 | -19.1 | -16.3 | -8.6 | -6.6 | -19.1 | -16.3 | -8.6 | -6.6 | -12.7 | 5.2 | 1.8 | -40.9% |

**Table 5**

| Direct dosing of coprocessed microparticles to the jejunum data | | | | | | | | | | | | |
|---|---|---|---|---|---|---|---|---|---|---|---|---|
| (Insulin (0.25mg/kg), Delivery Agent (37.5mg/kg)) | | | | | | | | | | | | |
| 1) | Insulin | | | | | | | | | | | |
| | | | | | | | | | | | | |
| jejunum | | | | | | | | | | | | |
| Time (min) | jej-1 | jej-2 | jej-3 | jej-4 | jej-5 | jej-6 | jej-7 | jej-8 | mean | SD | SEM | CV |
| 0 | 12.5 | 12.5 | 12.5 | 12.5 | 12.5 | 12.5 | 12.5 | 12.5 | 12.5 | 0.0 | 0.0 | 0.0% |
| 15 | 428.5 | 532.4 | 232.6 | 12.5 | 62.0 | 186.6 | 79.6 | 160.5 | 219.2 | 170.7 | 60.4 | 77.8% |
| 30 | 67.9 | 100.8 | 44.7 | 12.5 | 15.5 | 12.5 | 14.2 | 49.3 | 39.7 | 30.4 | 10.7 | 76.4% |
| 45 | 16.8 | 40.8 | 26.6 | 12.5 | 12.5 | 12.5 | 12.5 | 12.5 | 18.4 | 9.7 | 3.4 | 52.6% |
| 60 | 12.5 | 24.7 | 17.3 | 12.5 | 12.5 | 12.5 | 12.5 | 12.5 | 14.6 | 4.1 | 1.5 | 28.1% |
| 90 | 12.5 | 12.5 | 12.5 | 12.5 | 12.5 | 12.5 | 12.5 | 12.5 | 12.5 | 0.0 | 0.0 | 0.0% |
| AUC_{0→90} | 8261 | 10947 | 5229 | 1125 | 1913 | 3737 | 2157 | 3897 | 4658 | 3392 | 1199 | 72.8% |
| | | | | | | | | | | | | |

| 2) | Glucose | | | | | | | | | | | |
|---|---|---|---|---|---|---|---|---|---|---|---|---|
| | | | | | | | | | | | | |
| Change from base line | | | | Jejunum | | | | | | | | |
| Time (min) | #9 | #10 | #11 | #12 | #13 | #14 | #15 | #16 | Mean | SD | SEM | CV |
| 0 | 0 | 0 | 0 | 0 | 0 | 0 | 0 | 0 | 0 | 0 | 0.0 | |
| 15 | -29.7 | -20.5 | -35.8 | 1.3 | -28.9 | -18.2 | -25.9 | -25.6 | -22.9 | 11.2 | 3.9 | -48.9% |
| 30 | -52.2 | -54.5 | -41.9 | -1.3 | -50.0 | -60.3 | -36.8 | -52.8 | -43.7 | 18.7 | 6.5 | -42.8% |
| 45 | -63.6 | -65.3 | -43.8 | -0.7 | -56.8 | -69.2 | -40.8 | -59.7 | -59.0 | 22.3 | 7.8 | -37.8% |
| 60 | -56.9 | -69.4 | -33.8 | 11.6 | -56.8 | -62.1 | -27.2 | -55.7 | -41.9 | 28.1 | 10.6 | -67.1% |
| 90 | -63.9 | -59.7 | -26.9 | 8.3 | -50.0 | -31.8 | -11.8 | -28.4 | -28.6 | 22.7 | 8.5 | -79.4% |

### b. Results from Oral Gavage Experiments Using Tablet and Capsules

The glucose and insulin data from the three formulations tested are shown in Figures 4 and 5, respectively. The individual data are listed in Tables 6 to 7. The results from the direct dosing studies to the stomach and jejunum are included for comparison. The individual glucose and insulin data for the simple mix of insulin and delivery agent is shown in Table 8.

In the group of 10 rats that was dosed with capsules containing microparticles of coprocessed insulin and carrier, the average minimum glucose lowering was 70% from baseline at 30 minutes. One rat died at 15-30 minutes, likely due to hypoglycemia, six rats were rescued at 30 minutes with dextrose, an additional rat was rescued at 60 minutes, and two of the six that were rescued at 30 minutes died after 60 minutes. There were no signs of GI irritation or GI damage from the oral gavage procedure from necropsies of the rats after the experiment. The average minimum glucose lowering from tablets that contained the same amounts of insulin and carrier was 50%.

The corresponding insulin concentrations are shown in Figure 5. Insulin concentration is highest from the coprocessed microparticles in a capsule, followed by the tablet and the capsule of the simple mix.

In the oral gavage studies using capsules containing coprocessed microparticles, two (of 10) rats were found to exhibit high insulin absorption. Retainer samples were reassayed and insulin levels were approximately the same as those from the original samples, as shown in Table 6(3), shown above. Insulin levels with and without two high responders are shown in Figures 6 and 7, respectively. The individual and average insulin and glucose profiles from N=10 and N=8 are shown in Figures 13 to 16.

**Table 6**

| Oral gavage of tablets: Insulin (0.5mg/kg), Delivery Agent (75mg/kg) | | | | | | | | | | | | | | |
|---|---|---|---|---|---|---|---|---|---|---|---|---|---|---|
| 1) | Insulin | | | | | | | | | | | | | |
| Time (min) | Rat# 11 | #12 | #13 | #14 | #15 | #16 | #17 | #18 | #19 | #20 | mean | SD | SEM | CV |
| 0 | 12.5 | 12.5 | 12.5 | 12.5 | 12.5 | 12.5 | 12.5 | 12.5 | 12.8 | 12.5 | 12.5 | 0.1 | 0.0 | 0.8% |
| 15 | 468.8 | 162.1 | 700.1 | 12.5 | 1363.4 | 197.4 | 565.4 | 57.0 | 114.4 | 12.5 | 365.4 | 426.3 | 134.8 | 116.7% |
| 30 | 90.5 | 14.5 | 108.6 | 12.5 | 174.0 | 14.4 | 62.7 | 117.5 | 20.0 | 16.1 | 63.1 | 57.2 | 18.1 | 90.7% |
| 45 | 15.2 | 32.0 | 22.9 | 12.5 | 44.5 | 12.5 | 16.3 | 43.3 | 12.5 | 12.5 | 22.4 | 12.9 | 4.1 | 57.6% |
| 60 | 12.5 | 12.5 | 13.9 | 12.5 | 23.2 | 16.7 | 12.5 | 12.5 | 12.5 | 12.5 | 14.1 | 3.5 | 1.1 | 24.6% |
| 90 | 12.5 | 12.5 | 12.5 | 12.5 | 12.5 | 12.5 | 12.5 | 12.5 | 12.5 | 12.5 | 12.5 | 0.0 | 0.0 | 0.0% |
| **UC_{0→90}** | **9180** | **3692** | **13068** | **1125** | **24532** | **4022** | **10229** | **3830** | **2768** | **1179** | **7363** | **7259** | **2295** | **98.6%** |

| 2) | Glucose | | | | | | | | | | | | | |
|---|---|---|---|---|---|---|---|---|---|---|---|---|---|---|
| Change from baseline | | | | | | | | | | | | | | |
| Time (min) | Rat# 1 | #2 | #3 | #4 | #5 | #6 | #7 | #8 | #9 | #10 | mean | SD | SEM | CV |
| 0 | 0 | 0 | 0 | 0 | 0 | 0 | 0 | 0 | 0 | 0 | 0 | | 0.0 | 0.00% |
| 15 | -51.4 | -38.9 | -36.5 | -37.8 | -23.8 | -26.7 | 12.1 | 16.5 | 25.0 | 54.3 | -10.7 | 35.0 | 11.1 | -326.7% |
| 30 | -70.8 | -69.4 | -66.2 | -68.3 | -67.9 | -68.9 | -57.1 | -41.8 | -20.7 | 47.8 | -48.3 | 37.4 | 11.8 | -77.4% |
| 45 | -66.7 | -63.9 | -64.9 | -57.3 | -57.1 | -52.2 | -44.0 | -27.5 | -27.2 | 52.2 | -40.9 | 35.7 | 11.3 | -87.4% |
| 60 | -54.2 | -47.2 | -41.9 | -42.7 | -34.5 | -33.3 | -9.9 | -4.4 | -1.1 | 51.1 | -21.8 | 31.7 | 10.0 | -145.1% |
| 90 | -50.0 | -26.4 | -17.6 | -19.5 | -13.1 | 5.6 | 9.9 | 11.0 | 32.6 | 106.5 | 3.9 | 42.9 | 13.6 | 1099.9% |

**Table 7**

| Oral gavage of capsules containing coprocessed insulin and delivery agent data (Insulin (0.5mg/kg), Delivery Agent (75mg/kg)) | | | | | | | | | | | | | | |
|---|---|---|---|---|---|---|---|---|---|---|---|---|---|---|
| 1) | Insulin | | | | | | | | | | | | | |
| Time (min) | Rat# 11 | #12 | #13 | #14 | #15 | #16 | #17 | #18 | #19 | #20 | mean | SD | SEM | CV |
| 0 | 12.5 | 12.5 | 12.5 | 12.5 | 12.5 | 12.5 | 12.5 | 12.5 | 12.5 | 12.5 | 0.0 | 0.0 | 0.0 | 0.0% |
| 15 | 461.1 | 35.2 | 400.0 | 1080.6 | 143.1 | 36.7 | 7970.5 | 1611.3 | 369.8 | 922.5 | 1303.1 | 2396.7 | 757.9 | 183.9% |
| 30 | 244.0 | 120.0 | | 3268.4 | 35.3 | 32.7 | 5915.1 | 201.0 | 150.9 | 240.3 | 1134.2 | 2070.4 | 654.7 | 182.5% |
| 45 | 26.9 | 13.7 | | 3132.7 | 18.9 | 201.2 | 3309.5 | 58.7 | 12.5 | 38.3 | 756.9 | 1399.0 | 442.4 | 184.8% |
| 60 | 13.8 | 12.5 | | 2129.7 | 12.5 | 28.7 | 3693.9 | 16.0 | 12.5 | 12.5 | 659.1 | 1335.7 | 422.4 | 202.6% |
| 90 | 12.5 | 12.5 | | 984.9 | 12.5 | 12.5 | | | 12.5 | 12.5 | 151.4 | 367.5 | 116.2 | 242.7% |
| C_{0→90} (n=9) | 11752 | 3096 | | 175011 | 3522 | 4986 | 285725 | 28279 | 8561 | 18579 | 59946 | 100827 | 33609 | 168.2% |
| C_{0→90} (n=7) | 11752 | 3096 | | | 3522 | 4986 | | 28279 | 8561 | 18579 | 11254 | 9279 | 3507 | 82.4% |

| 2) | Glucose | | | | | | | | | | | | | |
|---|---|---|---|---|---|---|---|---|---|---|---|---|---|---|
| Time (min) | #11 | #12 | #13 | #14 | #15 | #16 | #17 | #18 | #19 | #20 | mean | SD | SEM | CV |
| 0 | 0 | 0 | 0 | 0 | 0 | 0 | 0 | 0 | 0 | 0 | 0 | 0 | 0.0 | 0.0% |
| 15 | -27.9 | 41.6 | -32.5 | -30.4 | -23.3 | 19.8 | -53.9 | -5.5 | -19.6 | -25.3 | -15.7 | 27.7 | 9.8 | -176.3% |
| 30 | -79.0 | -17.5 | | -76.6 | -64.0 | -40.1 | -78.0 | -77.0 | -63.8 | -77.0 | -63.7 | 21.4 | 7.5 | -33.5% |
| 45 | -42.1 | -46.4 | | -62.6 | -36.7 | -66.7 | -53.9 | -4.9 | 10.9 | -50.0 | -39.2 | 25.9 | 9.1 | -66.2% |
| 60 | 3.2 | -34.9 | | -61.4 | -13.3 | -75.3 | -70.3 | -7.2 | 64.5 | -48.3 | -30.8 | 45.9 | 16.2 | -149.0% |
| 90 | 68.4 | -7.2 | | -62.6 | 20.7 | 92.0 | | | 105.1 | -8.6 | 29.3 | 70.0 | 24.7 | 239.0% |

| | 3) | Reassay insulin levels of Rats 14 and 17 | | | | | | | | | | | | |
|---|---|---|---|---|---|---|---|---|---|---|---|---|---|---|
| | Rat #14 | | | | | | | | | | | | | |
| | Time (min) | | | | Insulin level (µU/ml), reassay | | | | Insulin level (µU/ml), original | | | | | |
| | 0 | | | | 12.5 | | | | 12.5 | | | | | |
| | 15 | | | | 1020.8 | | | | 1080.6 | | | | | |
| | 30 | | | | 3018.0 | | | | 3268.4 | | | | | |
| | 45 | | | | 2590.5 | | | | 3132.7 | | | | | |
| | 60 | | | | 1714.3 | | | | 2129.7 | | | | | |
| | 90 | | | | 996.9 | | | | 984.9 | | | | | |
| | | | | | | | | | | | | | | |

| | Rat #17 | | | | | | | | | | | | | |
|---|---|---|---|---|---|---|---|---|---|---|---|---|---|---|
| | Time (min) | | | | Insulin level (µU/ml), reassay | | | | Insulin level (µU/ml), original | | | | | |
| | 0 | | | | 12.5 | | | | 12.5 | | | | | |
| | 15 | | | | 7409.9 | | | | 7970.5 | | | | | |
| | 30 | | | | 6281.1 | | | | 5915.1 | | | | | |
| | 45 | | | | 2794.8 | | | | 3309.5 | | | | | |
| | 60 | | | | 2906.4 | | | | 3693.9 | | | | | |
| | 90 | | | | NS | | | | NS | | | | | |
| | | | | | | | | | | | | | | |

**Table 8**

| Oral gavage of capsules containing containing a simple mix of insulin and delivery agent data (Insulin (0.5mg/kg), Delivery Agent (75mg/kg)) | | | | | | | | | | | | | | |
|---|---|---|---|---|---|---|---|---|---|---|---|---|---|---|
| 1) | Insulin | | | | | | | | | | | | | |
| time | rat # 1 | #2 | #3 | #4 | #5 | #6 | #7 | #8 | #9 | #10 | mean | SD | SE | CV |
| 0 | 12.5 | 12.5 | 12.5 | 12.5 | 12.5 | 12.5 | 12.5 | 12.5 | 12.5 | 12.5 | 12.5 | 0 | 0 | 0.0% |
| 15 | 41.4 | 230.9 | 58.3 | 110.7 | 82.2 | 12.5 | 12.5 | 12.5 | 14.9 | 25.2 | 60.1 | 68.8 | 21.8 | 114.4% |
| 30 | 12.5 | 49.6 | 12.5 | 12.5 | 12.5 | 12.5 | 12.5 | 12.5 | 12.5 | 12.5 | 16.2 | 11.7 | 3.7 | 72.3% |
| 45 | 12.5 | 12.5 | 12.5 | 12.5 | 12.5 | 12.5 | 12.5 | 12.5 | 12.5 | 12.5 | 12.5 | 0 | 0 | 0.0% |
| 60 | 12.5 | 12.5 | 12.5 | 12.5 | 12.5 | 12.5 | 12.5 | 12.5 | 12.5 | 12.5 | 12.5 | 0 | 0 | 0.0% |
| 90 | 12.5 | 12.5 | 12.5 | 12.5 | 12.5 | 12.5 | 12.5 | 12.5 | 12.5 | 12.5 | 12.5 | 0 | 0 | 0.0% |
| **AUC_{0→90}** | **1559** | **4958** | **1812** | **2598** | **2171** | **1125** | **1125** | **1125** | **1161** | **1316** | **1895** | **1189** | **376** | **62.7%** |

| 2) | Glucose | | | | | | | | | | | | | |
|---|---|---|---|---|---|---|---|---|---|---|---|---|---|---|
| Change from base line | | | | | | | | | | | | | | |
| Time (min) 1 | Rat# | #2 | #3 | #4 | #5 | #6 | #7 | #8 | #9 | #10 | mean | SD | SEM | CV |
| 0 | 0 | 0 | 0 | 0 | 0 | 0 | 0 | 0 | 0 | 0 | 0 | 0 | 0 | 0% |
| 15 | 7.7 | -32.3 | 3.4 | 9.3 | 14.5 | 58.7 | 34 | 1.7 | 51.7 | 1.7 | 15.0 | 26.7 | 8.4 | 177.7% |
| 30 | -38.2 | -78.5 | -46.1 | -42.9 | -47.2 | 28.7 | 45.7 | 54.6 | 2.7 | -17.9 | -13.9 | 44.9 | 14.1 | -322.4% |
| 45 | -13.9 | -22.6 | -20.8 | -34.8 | -38.9 | 20.4 | 42.1 | 44.8 | -0.7 | 11.0 | -1.3 | 30.1 | 9.5 | -2247.0% |
| 60 | -3.1 | 41.4 | -18.5 | -14.3 | -26.9 | 20.9 | 65.5 | 47.7 | 26.2 | 32.9 | 17.2 | 31.3 | 9.9 | 182.1% |
| 90 | 6.2 | 73.1 | 32.6 | 53.4 | | 40.1 | 43.1 | 51.7 | 39.6 | 30.6 | 41.2 | 18.4 | 5.8 | 44.6% |

### Example 2

### 1. Summary of Intravenous, Portal Vein and Subcutaneous Experiments

### a. Experiment

Intravenous, intraportal and subcutaneous dosing in rodents were conducted to estimate the absolute bioavailability, the absorption of insulin in the portal vein, and the bioavailability to relative subcutaneous administration. The data are summarized in Tables 9 to 11. The average insulin AUC_{0→∞}/Dose was 0.0093 min.kg/ml from intravenous dosing. This value was assumed to be constant in the estimates of absolute bioavailability.

**Table 9**

| | Insulin pharmacokinetics results from intravenous (IV) administration | | | | | | | |
|---|---|---|---|---|---|---|---|---|
| **Dose** | **Cₒ** | **kₑₗ** | **AUC_{0→∞}** | **AUC_{0→∞}/Dose** | **V_{d}** | | **C_{0→∞}/Dose** | **AUC_{0→∞}/Dose** |
| **(µg/kg**) | **(ng/ml)** | **(min⁻¹)** | **(min. ng/ml)** | **(min. kg/ml)** | **(ml/kg)** | | **(kg/ml)** | **(min. kg/ml)** |
| 2.5 | 15.79 | 0.89 | 17.74 | 0.0071 | 158 | | 0.0063 | 0.0071 |
| 2.5 | 7.48 | 0.71 | 10.56 | 0.0042 | 334 | | 0.0030 | 0.0042 |
| 2.5 | 13.36 | 0.84 | 15.87 | 0.0063 | 187 | | 0.0053 | 0.0063 |
| 2.5 | 12.78 | 0.72 | 17.73 | 0.0071 | 196 | | 0.0051 | 0.0071 |
| 2.5 | 11.26 | 0.72 | 15.75 | 0.0063 | 222 | | 0.0045 | 0.0063 |
| ***X*±*SD (n=5)*** | ***12.1* ± *2.8*** | ***0.78* ± *0.07*** | ***15.5* ± *2.6*** | ***0.0062* ± *0.0011*** | ***219* ± *68*** | | | |
| | | | | | | | | |
| 8 | 50.67 | 0.51 | 99.35 | 0.0124 | 157.88 | | 0.0063 | 0.0124 |
| 8 | 52.64 | 0.53 | 99.32 | 0.0124 | 151.98 | | 0.0066 | 0.0124 |
| 8 | 49.63 | 0.48 | 103.4 | 0.0129 | 161.19 | | 0.0062 | 0.0129 |
| ***X*±*SD (n=3)*** | ***51.0* ± *1.5*** | ***0.51* ± *0.03*** | ***100.7* ± 2.4** | ***0.0126* ± *0.0003*** | ***157* ± *5*** | | | |
| | | | | | | | | |
| 9 | 46.79 | 0.47 | 99.55 | 0.0111 | 192.35 | | 0.0052 | 0.0111 |
| 9 | 36.32 | 0.48 | 75.67 | 0.0084 | 247.80 | | 0.0040 | 0.0084 |
| 9 | 55.30 | 0.45 | 122.89 | 0.0137 | 162.75 | | 0.0061 | 0.0137 |
| ***X*±*SD (n=3)*** | ***46.1*± *9.5*** | ***0.47* ± *0.02*** | ***99.4* ± *23.6*** | ***0.0110*± *0.0026*** | ***201*± *43*** | | | |
| | | | | | | | | |
| | | | | | | Average | 0.0053 | 0.0093 |
| | | | | | | SD | 0.001133858 | 0.0033 |
| | | | | | | SEM | 0.000341871 | 0.00099 |

**Table 10**

| Insulin results from portal and systemic administrations | | | | |
|---|---|---|---|---|
| n | AUC-IP | AUC-JV | AUC ratio | FPE (fH) |
| 1 | 9.95 | 17.74 | 0.56 | 0.44 |
| 2 | 6.21 | 10.56 | 0.59 | 0.41 |
| 3 | 8.88 | 15.87 | 0.56 | 0.44 |
| 4 | 14.51 | 17.73 | 0.82 | 0.18 |
| 5 | 8.69 | 15.75 | 0.55 | 0.45 |
| Average | 9.65 | 15.53 | 0.62 | 0.38 |
| SD | 3.04 | 2.94 | 0.12 | 0.12 |

**Table 11**

| Insulin results from subcutaneous (SC) administration | | | | | | | | | | | | | | |
|---|---|---|---|---|---|---|---|---|---|---|---|---|---|---|
| **dy** # | **Insulin dose** | **Time** | **Insulin** | **SD** | **SE** | **Glucose** | **SD** | **SE** | **N** | **AUC₀**- **>T** | **Tₘₐₓ** | **Cₘₐₓ** | **AUC_{0→T}** | **AUC_{0→T} Dose** |
| | **(mg/kg)** | | **(uU/ml)** | | | **(mg/dl)** | | | | **(min. uU/ml)** | **(min)** | | **(min. ng/ml)** | **(min. kg/ml)** |
| 1 | 0.025 | 0 | 0.49 | 0.77 | 0.31 | 121 | 12.6 | 5.1 | 6 | 3640.8 | 15 | 76.1 | 140.0 | 0.005601 |
| | | 15 | 76.14 | 17.52 | 7.15 | 110 | 26.4 | 10.7 | 6 | | | | | |
| | | 30 | 54.34 | 21.92 | 8.95 | 90 | 31.4 | 12.8 | 6 | | | | | |
| | | 45 | 32.26 | 4.59 | 1.87 | 101 | 36.6 | 14.9 | 6 | | | | | |
| | | 60 | 21.61 | 4.29 | 1.75 | 102 | 30.4 | 12.4 | 6 | | | | | |
| | | 120 | 5.61 | 2.93 | 1.19 | 130 | 37.0 | 15.1 | 6 | | | | | |
| | | 180 | 1.64 | 1.82 | 0.74 | 194 | 54.7 | 22.3 | 6 | | | | | |
| 2 | 0.025 | 0 | 2.15 | 2.37 | 0.96 | 116 | 12.9 | 5.2 | 6 | 4234.4 | 15 | 79.5 | 162.9 | 0.006514 |
| | | 15 | 79.54 | 24.22 | 9.88 | 103 | 8.3 | 3.3 | 6 | | | | | |
| | | 30 | 53.03 | 20.27 | 8.27 | 90 | 17.4 | 7.1 | 6 | | | | | |
| | | 45 | 45.53 | 23.98 | 9.79 | 100 | 26.2 | 10.7 | 6 | | | | | |
| | | 60 | 23.48 | 21.47 | 8.76 | 120 | 23.6 | 9.6 | 6 | | | | | |
| | | 120 | 9.36 | 9.60 | 3.92 | 196 | 45.6 | 18.6 | 6 | | | | | |
| | | 180 | 3.48 | 5.23 | 2.13 | 188 | 45.1 | 18.4 | 6 | | | | | |
| 3 | 0.05 | 0 | 1.51 | 1.59 | 0.71 | 83 | 6.9 | 3.1 | 5 | 7230.0 | 15 | 98.8 | 278.1 | 0.005562 |
| | | 15 | 98.85 | 14.16 | 6.33 | 66 | 17.1 | 7.6 | 5 | | | | | |
| | | 30 | 73.59 | 25.49 | 11.40 | 60 | 21.4 | 9.5 | 5 | | | | | |
| | | 45 | 61.37 | 21.56 | 9.64 | 61 | 27.2 | 12.1 | 5 | | | | | |
| | | 60 | 58.20 | 32.09 | 14.35 | 73 | 24.4 | 10.9 | 5 | | | | | |
| | | 120 | 21.34 | 17.43 | 7.79 | 98 | 21.6 | 9.7 | 5 | | | | | |
| | | 180 | 8.30 | 7.49 | 3.35 | 105 | 30.7 | 13.7 | 5 | | | | | |
| 4 | 0.05 | 0 | 0.37 | 0.90 | 0.36 | 73 | 4.8 | 1.9 | 6 | 6407.7 | 15 | 103.4 | 246.5 | 0.004929 |
| | | 15 | 103.42 | 25.17 | 10.27 | 66 | 6.9 | 2.8 | 6 | | | | | |
| | | 30 | 81.64 | 24.94 | 10.18 | 59 | 10.5 | 4.3 | 6 | | | | | |
| | | 45 | 68.91 | 20.41 | 8.33 | 64 | 9.0 | 3.6 | 6 | | | | | |
| | | 60 | 58.58 | 21.87 | 8.93 | 72 | 7.0 | 2.8 | 6 | | | | | |
| | | 90 | 32.01 | 9.92 | 4.05 | 88 | 14.0 | 5.7 | 6 | | | | | |
| | | 120 | 21.15 | 9.42 | 3.84 | 110 | 17.0 | 6.9 | 6 | | | | | |
| 5 | 0.05 | 0 | 0 | 0 | 0 | 60 | 5.1 | 2.2 | 5 | 1317.0 | 15 | 34.9 | 50.7 | 0.001013 |
| | | 15 | 34.90 | 46.01 | 20.57 | 42 | 6.1 | 2.7 | 5 | | | | | |
| | | 30 | 9.71 | 18.15 | 8.12 | 38 | 6.9 | 3.1 | 5 | | | | | |
| | | 45 | 16.57 | 37.04 | 16.56 | 42 | 9.8 | 4.4 | 5 | | | | | |
| | | 60 | 10.46 | 20.05 | 8.96 | 48 | 17.6 | 7.8 | 5 | | | | | |
| | | 90 | 2.94 | 4.25 | 1.90 | 52 | 25.2 | 11.3 | 5 | | | | | |
| | | 120 | 5.05 | 6.91 | 3.09 | 66 | 41.8 | 18.7 | 5 | | | | | |
| 6 | 0.05 | 0 | 12.80 | 18.01 | 7.35 | 68 | 5.0 | 2.0 | 6 | 9937.5 | 15 | 191.7 | 382.2 | 0.007644 |
| | | 15 | 191.67 | 100.40 | 40.98 | 40 | 6.2 | 2.5 | 6 | | | | | |
| | | 30 | 119.71 | 79.53 | 35.56 | 39 | 2.5 | 1.1 | 5 | | | | | |
| | | 45 | 121.33 | 118.89 | 48.53 | 36 | 7.1 | 2.9 | 6 | | | | | |
| | | 60 | 74.65 | 57.97 | 23.66 | 32 | 4.3 | 1.7 | 6 | | | | | |
| | | 90 | 42.88 | 37.77 | 15.42 | 35 | 5.5 | 2.2 | 6 | | | | | |
| | | 120 | 25.65 | 17.59 | 7.18 | 39 | 9.2 | 3.7 | 6 | | | | | |
| 7 | 0.05 | 0 | 5.85 | 3.11 | 1.27 | 65 | 8.7 | 3.5 | 6 | 7711.1 | 30 | 126.0 | 296.6 | 0.005932 |
| | | 15 | 90.29 | 73.70 | 30.08 | 45 | 7.2 | 2.9 | 6 | | | | | |
| | | 30 | 125.96 | 107.54 | 43.90 | 39 | 8.9 | 3.6 | 6 | | | | | |
| | | 45 | 67.83 | 95.55 | 39.01 | 40 | 10.9 | 4.4 | 6 | | | | | |
| | | 60 | 78.19 | 105.69 | 43.14 | 42 | 13.2 | 5.4 | 6 | | | | | |
| | | 90 | 45.77 | 49.52 | 20.22 | 42 | 18.7 | 7.6 | 6 | | | | | |
| | | 120 | 18.24 | 13.53 | 5.52 | 50 | 26.2 | 10.7 | 6 | | | | | |
| 8 | 0.05 | 0 | 2.40 | 5.88 | 2.40 | 70 | 4.8 | 1.9 | 6 | 5303.3 | 15 | 131.7 | 204.0 | 0.004079 |
| | | 15 | 131.71 | 131.15 | 53.54 | 59 | 6.8 | 3.0 | 6 | | | | | |
| | | 30 | 70.90 | 45.95 | 18.76 | 64 | 12.1 | 4.9 | 6 | | | | | |
| | | 45 | 66.28 | 42.41 | 17.31 | 69 | 16.5 | 6.7 | 6 | | | | | |
| | | 60 | 0.59 | 1.27 | 0.52 | 73 | 13.1 | 5.3 | 6 | | | | | |
| | | 90 | 33.96 | 14.99 | 6.12 | 89 | 17.8 | 7.2 | 6 | | | | | |
| | | 120 | 14.64 | 9.35 | 3.82 | 106 | 18.6 | 7.6 | 6 | | | | | |
| | | | | | | | | | | | | | **Avg AUC_{0->T}/Dose** | 0.005159 |
| | | | | | | | | | | | | | **(min. kg/ml)** | |

### b. Results

The ratio of systemic to portal insulin was found to be approximately 0.62 (calculated from data in Table 10). Hence, the bioavailability in the portal vein can be calculated by dividing the absolute bioavailability by 0.62. The portal bioavailability provides an estimate of drug absorption from oral delivery. The average insulin AUC_{0->t}/Dose was 0.00516 min.kg/ml from subcutaneous dosing. This value is used to estimate bioavailability relative to subcutaneous. With the exception of the intravenous data, all AUC were calculated from t=0 to the last sampling point (i.e. AUC_{0->t}).

In the rat model, these results from intraportal administration suggest that the maximum absolute bioavailability of insulin is approximately 60% from oral delivery or by any other means of 100% GI absorption of insulin into the portal vein. Secondly, the absolute bioavailability from SC is approximately 56%.

The estimates of bioavailability (absolute bioavailability, portal bioavailability, relative bioavailability to subcutaneous, and relative portal bioavailability to subcutaneous) are summarized in Figures 8 to 12, and in Table 12. The estimated absolute bioavailability from in situ dosing to the stomach and the jejunum are shown in Figure 8. The values of bioavailability were 5 % when dosed in the stomach and 18 % when dosed in the jejunum from microparticles containing coprocessed insulin (0.5 mg/kg) and delivery agent (75 mg/kg).

The estimated absolute bioavailability from the tablet and capsule formulations dosed by oral gavage in rats are shown in Figure 9 using formulations containing 0.5 mg/kg insulin and 75 mg/kg delivery agent. The values of bioavailability were 6% when dosed from tablets, and 1.6% when dosed from capsules containing a simple mix of insulin and carrier.

**Table 12**

| **Estimates of bioavailability** | | | | | | | | | |
|---|---|---|---|---|---|---|---|---|---|
| | **AUC_{0->T}** | **Tₘₐₓ** | **Cₘₐₓ** | **AUC_{0->T}** | **AU C_{0->T} /Dose** | **BA** | **BAₚₒᵣₜₐₗ** | **Rel BA** | **Rel BAₚₒᵣₜₐₗ** |
| | **(min. uU/ml)** | **(min)** | **(uU/ml)** | **(min. ng/ml)** | **(min. kg/ml)** | **(%)** | **(%)** | **(%)** | **(%)** |
| | | | | | | | (IP/JV=0.62) | **[AUC_{0->T} /Dose]sc=0.0052** | **[AUC_{0->T/} Dose]sc=0.0052** |
| SC | | | | | 0.0052 | 55.67 | | | |
| **Insulin (0.5mg/kg) + Delivery Agent (75mg/kg)** | | | | | | | | | |
| **Stomach** | | | | | | | | | |
| | 4778.70 | 15 | 114.50 | 183.80 | 3.68E-04 | 3.97 | 6.40 | 7.12 | 11.49 |
| | 2130.75 | 15 | 72.75 | 81.95 | 1.64E-04 | 1.77 | 2.85 | 3.18 | 5.12 |
| | 18958.58 | 45 | 893.86 | 729.18 | 1.46E-03 | 15.74 | 25.38 | 28.27 | 45.59 |
| | 1126.65 | 15 | 12.61 | 43.33 | 8.67E-05 | 0.94 | 1.51 | 1.68 | 2.71 |
| | 14425.95 | 45 | 212.73 | 554.84 | 1.11E-03 | 11.97 | 19.31 | 21.51 | 34.69 |
| | 4000.73 | 60 | 140.31 | 153.87 | 3.08E-04 | 3.32 | 5.36 | 5.96 | 9.62 |
| | 2793.45 | 15 | 118.66 | 107.44 | 2.15E-04 | 2.32 | 3.74 | 4.16 | 6.72 |
| | 5046.23 | 15 | 158.50 | 194.09 | 3.88E-04 | 4.19 | 6.76 | 7.52 | 12.13 |
| | | | | | | | | | |
| **Mean** | 6657.63 | 28.13 | 215.49 | 256.06 | 5.12E-04 | 5.53 | 8.91 | 9.93 | 16.01 |
| **SD** | 6446.07 | 18.70 | 280.36 | 247.93 | 4.96E-04 | 5.35 | 8.63 | 9.61 | 15.50 |
| **SE** | 2279.03 | 6.61 | 99.12 | 87.65 | 1.75E-04 | 1.89 | 3.05 | 3.40 | 5.48 |
| **CV (%)** | 96.82 | 66.48 | 130.10 | 96.82 | 9.68E+01 | 96.82 | 96.82 | 96.82 | 96.82 |
| | | | | | | | | | |
| **Jejunum** | | | | | | | | | |
| | 13504.88 | 15 | 413.23 | 519.42 | 1.04E-03 | 11.21 | 18.08 | 20.14 | 32.48 |
| | 21156.68 | 15 | 1193.25 | 813.72 | 1.63E-03 | 17.56 | 28.32 | 31.54 | 50.88 |
| | 11967.90 | 15 | 669.38 | 460.30 | 9.21E-04 | 9.93 | 16.02 | 17.84 | 28.78 |
| | 19689.45 | 15 | 1177.47 | 757.29 | 1.51E-03 | 16.34 | 26.36 | 29.36 | 47.35 |
| | 46001.85 | 15 | 2270.55 | 1769.30 | 3.54E-03 | 38.18 | 61.59 | 68.59 | 110.62 |
| | 11101.35 | 15 | 228.90 | 426.98 | 8.54E-04 | 9.21 | 14.86 | 16.55 | 26.70 |
| | 39190.05 | 15 | 954.35 | 1507.31 | 3.01E-03 | 32.53 | 52.47 | 58.43 | 94.24 |
| | 7234.05 | 15 | 374.88 | 278.23 | 5.56E-04 | 6.00 | 9.68 | 10.79 | 17.40 |
| | | | | | | | | | |
| **Mean** | 21230.78 | 15 | 910.25 | 816.57 | 1.63E-03 | 17.62 | 28.42 | 31.65 | 51.05 |
| **SD** | 14053.61 | 0 | 661.14 | 540.52 | 1.08E-03 | 11.66 | 18.81 | 20.95 | 33.80 |
| **SE** | 4968.70 | 0 | 233.75 | 191.10 | 3.82E-04 | 4.12 | 6.65 | 7.41 | 11.95 |
| **CV (%)** | 66.19 | 0 | 72.63 | 66.19 | 6.62E+01 | 66.19 | 66.19 | 66.19 | 66.19 |
| | | | | | | | | | |
| **Tablet** | | | | | | | | | |
| | 9181.20 | 15 | 468.81 | 353.12 | 7.06E-04 | 7.62 | 12.29 | 13.69 | 22.08 |
| | 3692.64 | 15 | 162.11 | 142.02 | 2.84E-04 | 3.07 | 4.94 | 5.51 | 8.88 |
| | 13068.89 | 15 | 700.10 | 502.65 | 1.01E-03 | 10.85 | 17.50 | 19.48 | 31.43 |
| | 1125.00 | 0 | 12.50 | 43.27 | 8.65E-05 | 0.93 | 1.51 | 1.68 | 2.71 |
| | 24533.57 | 15 | 1363.43 | 943.60 | 1.89E-03 | 20.36 | 32.84 | 36.58 | 59.00 |
| | 4022.33 | 15 | 197.39 | 154.70 | 3.09E-04 | 3.34 | 5.38 | 6.00 | 9.67 |
| | 10228.04 | 15 | 565.39 | 393.39 | 7.87E-04 | 8.49 | 13.69 | 15.25 | 24.60 |
| | 3830.06 | 30 | 117.47 | 147.31 | 2.95E-04 | 3.18 | 5.13 | 5.71 | 9.21 |
| | 2767.70 | 15 | 114.35 | 106.45 | 2.13E-04 | 2.30 | 3.71 | 4.13 | 6.66 |
| | 1178.43 | 30 | 16.06 | 45.32 | 9.06E-05 | 0.98 | 1.58 | 1.76 | 2.83 |
| | | | | | | | | | |
| **Mean** | 7362.78 | 16.50 | 371.76 | 283.18 | 5.66E-04 | 6.11 | 9.86 | 10.98 | 17.71 |
| **SD** | 7669.89 | 9.01 | 445.61 | 295.00 | 5.90E-04 | 6.37 | 10.27 | 11.44 | 18.44 |
| **SE** | 2425.43 | 2.85 | 140.91 | 93.29 | 1.87E-04 | 2.01 | 3.25 | 3.62 | 5.83 |
| **CV (%)** | 104.17 | 54.63 | 119.86 | 104.17 | 1.04E+02 | 104.17 | 104.17 | 104.17 | 104.17 |
| | | | | | | | | | |
| **Capsule (co-dried)** | | | | | | | | | |
| **(N=9)** | 11570.64 | 15 | 461.06 | 445.02 | 8.90E-04 | 9.60 | 15.49 | 17.25 | 27.82 |
| | 3096.24 | 30 | 120.02 | 119.09 | 2.38E-04 | 2.57 | 4.15 | 4.62 | 7.45 |
| | 175011.18 | 30 | 3268.40 | 6731.20 | 1.35E-02 | 145.27 | 234.30 | 260.93 | 420.86 |
| | 3523.46 | 15 | 143.14 | 135.52 | 2.71E-04 | 2.92 | 4.72 | 5.25 | 8.47 |
| | 4988.01 | 45 | 201.24 | 191.85 | 3.84E-04 | 4.14 | 6.68 | 7.44 | 11.99 |
| | 285725.26 | 15 7 | 7970.50 | 10989.43 | 2.20E-02 2: | 237.16 | 382.52 | 426.00 | 687.10 |
| | 28278.49 | 15 1 | 1611.31 | 1087.63 | 2.18E-03 | 23.47 | 37.86 | 42.16 | 68.00 |
| | 8559.93 | 15 | 369.78 | 329.23 | 6.58E-04 | 7.11 | 11.46 | 12.76 | 20.58 |
| | 18578.54 | 15 | 922.50 | 714.56 | 1.43E-03 | 15.42 | 24.87 | 27.70 | 44.68 |
| | | | | | | | | | |
| **Mean** | 59925.75 | 21.67 | 1674.22 | 2304.84 | 0.00 | 49.74 | 80.23 | 89.35 | 144.11 |
| **SD** | 100838.27 | 10.90 | 2570.42 | 3878.39 | 0.01 | 83.70 | 135.00 | 150.34 | 242.49 80.83 |
| **SE** | 33612.76 | 3.63 | 856.81 | 1292.80 | 0.00 | 27.90 | 45.00 | 50.11 | |
| **CV (%)** | 168.27 | 50.29 | 153.53 | 168.27 | 168.27 | 168.27 | 168.27 | 168.27 | 168.27 |
| **Capsule (co-dried)** | | | | | | | | | |
| **(N=7)** | 11570.64 | 15 | 461.06 | 445.02 | 8.90E-04 | 9.60 | 15.49 | 17.25 | 27.82 |
| | 3096.24 | 30 | 120.02 | 119.09 | 2.38E-04 | 2.57 | 4.15 | 4.62 | 7.45 |
| | 3523.46 | 15 | 143.14 | 135.52 | 2.71E-04 | 2.92 | 4.72 | 5.25 | 8.47 |
| | 4988.01 | 45 | 201.24 | 191.85 | 3.84E-04 | 4.14 | 6.68 | 7.44 | 11.99 |
| | 28278.49 | 15 | 1611.31 | 1087.63 | 2.18E-03 | 23.47 | 37.86 | 42.16 | 68.00 |
| | 8559.93 | 15 | 369.78 | 329.23 | 6.58E-04 | 7.11 | 11.46 | 12.76 | 20.58 |
| | 18578.54 | 15 | 922.50 | 714.56 | 1.43E-03 | 15.42 | 24.87 | 27.70 | 44.68 |
| | | | | | | | | | |
| **Mean** | 11227.90 | 21.43 | 547.01 | 431.84 | 0.00 | 9.32 | 15.03 | 16.74 | 27.00 |
| **SD** | 9277.29 | 11.80 | 544.29 | 356.82 | 0.00 | 7.70 | 12.42 | 13.83 | 22.31 |
| **SE** | 3506.49 | 4.46 | 205.72 | 134.86 | 0.00 | 2.91 | 4.69 | 5.23 | 8.43 |
| **CV (%)** | 82.63 | 55.08 | 99.50 | 82.63 | 82.63 | 82.63 | 82.63 | 82.63 | 82.63 |
| **Capsule (simple mix)** | | | | | | | | | |
| | 1558.08 | 15 | 41.37 | 59.93 | 1.20E-04 | 1.29 | 2.09 | 2.32 | 3.75 |
| | 4956.66 | 15 | 230.89 | 190.64 | 3.81E-04 | 4.11 | 6.64 | 7.39 | 11.92 |
| | 1812.60 | 15 | 58.34 | 69.72 | 1.39E-04 | 1.50 | 2.43 | 2.70 | 4.36 |
| | 2598.35 | 15 | 110.72 | 99.94 | 2.0OE-04 | 2.16 | 3.48 | 3.87 | 6.25 |
| | 2171.06 | 15 | 82.24 | 83.50 | 1.67E-04 | 1.80 | 2.91 | 3.24 | 5.22 |
| | 1125.00 | 0 | 12.50 | 43.27 | 8.65E-05 | 0.93 | 1.51 | 1.68 | 2.71 |
| | 1125.00 | 0 | 12.50 | 43.27 | 8.65E-05 | 0.93 | 1.51 | 1.68 | 2.71 |
| | 1125.00 | 0 | 12.50 | 43.27 | 8.65E-05 | 0.93 | 1.51 | 1.68 | 2.71 |
| | 1161.65 | 15 | 14.94 | 44.68 | 8.94E-05 | 0.96 | 1.56 | 1.73 | 2.79 |
| | 1315.97 | 15 | 25.23 | 50.61 | 1.01E-04 | 1.09 | 1.76 | 1.96 | 3.16 |
| | | | | | | | | | |
| **Mean** | 1894.94 | 10.50 | 60.12 | 72.88 | 1.46E-04 | 1.57 | 2.54 | 2.83 | 4.56 |
| **SD** | 1188.69 | 7.25 | 68.82 | 45.72 | 9.14E-05 | 0.99 | 1.59 | 1.77 | 2.86 |
| **SE** | 375.90 | 2.29 | 21.76 | 14.46 | 2.89E-05 | 0.31 | 0.50 | 0.56 | 0.90 |
| **CV (%)** | 62.73 | 69.01 | 114.47 | 62.73 | 6.27E+01 | 62.73 | 62.73 | 62.73 | 62.73 |
| | | | | | | | | | |
| **Insulin (0.25mg/kg) + Delivery Agent (37.5mg/kg) Stomach** | | | | | | | | | |
| | 1669.5D | 45 | 48.80 | 64.21 | 2.57E-04 | 2.77 | 4.47 | 4.98 | 8.03 |
| | 1249.50 | 15 | 20.80 | 48.06 | 1.92E-04 | 2.07 | 3.35 | 3.73 | 6.01 |
| | 1373.25 | 0 | 41.20 | 52.82 | 2.11E-04 | 2.28 | 3.68 | 4.09 | 6.60 |
| | 1192.50 | 0 | 21.50 | 45.87 | 1.83E-04 | 1.98 | 3.19 | 3.56 | 5.74 |
| | 1125.00 | 0 | 12.50 | 43.27 | 1.73E-04 | 1.87 | 3.01 | 3.35 | 5.41 |
| | 1125.00 | 0 | 12.50 | 43.27 | 1.73E-04 | 1.87 | 3.01 | 3.35 | 5.41 |
| | 1333.22 | 15 | 26.38 | 51.28 | 2.05E-04 | 2.21 | 3.57 | 3.98 | 6.41 |
| | 1125.00 | 0 | 12.50 | 43.27 | 1.73E-04 | 1.87 | 3.01 | 3.35 | 5.41 |
| | | | | | | | | | |
| **Mean** | 1274.12 | 9.38 | 24.52 | 49.00 | 1.96E-04 | 2.12 | 3.41 | 3.80 | 6.13 |
| **SD** | 186.56 | 15.91 | 13.77 | 7.18 | 2.87E-05 | 0.31 | 0.50 | 0.56 | 0.90 |
| **SE** | 65.96 | 5.63 | 4.87 | 2.54 | 1.01E-05 | 0.11 | 0.18 | 0.20 | 0.32 |
| **CV (%)** | 14.64 | 169.71 | 56.16 | 14.64 | 1.46E+01 | 14.64 | 14.64 | 14.64 | 14.64 |
| | | | | | | | | | |
| **Jejunum** | 8260.50 | 15 | 428.50 | 317.71 | 1.27E-03 | 13.71 | 22.12 | 24.63 | 39.73 |
| | 10947.00 | 15 | 532.40 | 421.04 | 1.68E-03 | 18.17 | 29.31 | 32.64 | 52.65 |
| | 5229.00 | 15 | 232.60 | 201.12 | 8.04E-04 | 8.68 | 14.00 | 15.59 | 25.15 |
| | 1125.00 | 0 | 12.50 | 43.27 | 1.73E-04 | 1.87 | 3.01 | 3.35 | 5.41 |
| | 1910.94 | 15 | 61.95 | 73.50 | 2.94E-04 | 3.17 | 5.12 | 5.70 | 9.19 |
| | 3735.93 | 15 | 186.56 | 143.69 | 5.75E-04 | 6.20 | 10.00 | 11.14 | 17.97 |
| | 2157.20 | 15 | 79.60 | 82.97 | 3.32E-04 | 3.58 | 5.78 | 6.43 | 10.38 |
| | 3897.03 | 15 | 160.54 | 149.89 | 6.00E-04 | 6.47 | 10.43 | 11.62 | 18.74 |
| | | | | | | | | | |
| **Mean** | 4657.82 | 13.13 | 211.83 | 179.15 | 7.17E-04 | 7.73 | 12.47 | 13.89 | 22.40 |
| **SD** | 3392.58 | 5.30 | 182.48 | 130.48 | 5.22E-04 | 5.63 | 9.08 | 10.12 | 16.32 |
| **SE** | 1199.46 | 1.88 | 64.52 | 46.13 | 1.85E-04 | 1.99 | 3.21 | 3.58 | 5.77 |
| **CV (%)** | 72.84 | 40.41 | 86.14 | 72.84 | 7.28E+01 | 72.84 | 72.84 | 72.84 | 72.84 |

### Example 3

### Insulin and 4-CNAB Stability in Simulated Gastric Fluid

The stability of insulin in simulated gastric fluid (SGF) was evaluated in the presence and absence of 4-CNAB. Solutions were prepared containing insulin (1 mg/ml) with and without monosodium 4-CNAB (1 mg/ml).

The SGF was prepared with and without pepsin, a gastric enzyme. SGF pH 1.2 was prepared as per the USP NF 26 guidelines. 2 g sodium chloride and 3.2 g of pepsin were weighed and added to a suitable container, and deionized water was added to reach one liter in volume. If necessary, the pH was adjusted to 1.2 by addition of concentrated HCl or NaOH. A second SGF solution omitting the pepsin was also prepared.

Four 50 ml samples of SGF (two with pepsin and two without) were placed into a jacketed vessel connected to a circulating water bath set at 37°C. The solutions were stirred with magnetic stir bars for ten minutes to allow the solutions to reach 37°C and reach thermal equilibrium. 50 mg of 4-CNAB was added to one of the samples containing pepsin and one of the samples without pepsin, and the solutions were stirred for a few minutes to allow the 4-CNAB to dissolve. 50 mg of insulin was added to the each of the samples. After dissolution of the insulin, samples of the solutions were taken at predetermined time intervals, filtered, and immediately assayed by HPLC for insulin and 4-CNAB content. The first sample withdrawn after all the insulin was dissolved was considered to have been drawn at time zero (0). The results are shown in table 13.

**Table 13**

| | Insulin with 4-CNAB and Enzymes (pepsin) | | Insulin without 4-CNAB and with Enzymes (pepsin) | Insulin without 4-CNAB and without Enzymes | Insulin with 4-CNAB and no Enzymes | |
|---|---|---|---|---|---|---|
| Time | Insulin % of theoretical | 4-CNAB % of theoretical | Insulin % of theoretical | Insulin % of theoretical | Insulin % of theoretical | 4-CNAB % of theoretical |
| 0 minutes | 3.0 | 105.6 | 3.0 | 99.8 | 100.8 | 95.3 |
| 10 minutes | | | | 100.4 | 103.9 | 95.7 |
| 20 minutes | | | | 100.6 | 103.8 | 99.6 |
| 30 minutes | | | | 100.1 | 105.7 | 99.2 |
| 1 h | | | | 99.4 | 97.8 | 94.8 |
| 2 h | | | | 99.0 | 102.2 | 95.8 |
| 24 h | | | | 0 | 0 | 91.2 |

The term " % of theoretical" as used herein, means the percent of the concentration (mg/mL) of withdrawn solution at the time-point the sample was taken as compared to the theoretical concentration (mg/mL) of the measuring component for experiment. The standard of deviation for the HPLC analysis is ±5 %. These results show that insulin is unstable in SGF containing pepsin, since only 3.0% of the insulin remained at the first sampling point (97% of the insulin was degraded), while insulin is stable at least up to 2 hours in SGF without pepsin.

### Example 4

### Stability of Insulin in Simulated Intestinal Fluid

The stability of insulin in simulated intestinal fluid (SIF) was evaluated in the presence and absence of 4-CNAB.

The SIF solutions were prepared with and without pancreatic enzyme. SIF pH 7.5 was prepared as per the USP NF 26 guidelines. SIF was prepared by addition of 6:8 g monobasic potassium phosphate and 10 g of pancreatin into a suitable vessel, and deionized water was added to reach a total volume of one liter. If necessary, the pH was adjusted to 7.5 by addition of 0.2 N sodium hydroxide. A second SIF solution omitting the pancreatin, an intestinal enzyme, was also prepared.

Four 50 ml samples of SIF (two with pancreatin and two without) were placed into a jacketed vessel connected to a circulating water bath set at 37°C. The solutions were stirred with magnetic stir bars for ten minutes to allow the solutions to reach 37°C and reach thermal equilibrium. 50 mg of 4-CNAB was added to one of the samples containing pepsin and one of the samples without pepsin, and the solutions were stirred for a few minutes to allow the 4-CNAB to dissolve. 50 mg of insulin was added to the each of the samples. After dissolution of the insulin, samples of the solutions were taken at predetermined time intervals, and immediately assayed by HPLC for insulin and 4-CNAB content. The results are shown in table 14.

**Table 14**

| | Insulin with 4-CNAB and Enzymes (pancreatin) | | Insulin without 4-CNAB or Enzymes | Insulin without 4-CNAB and with Enzymes (pancreatin) | Insulin with 4-CNAB and no Enzymes | |
|---|---|---|---|---|---|---|
| Time | Insulin % of theoretical | 4-CNAB % of theoretical | Insulin % of theoretical | 4-CNAB % of theoretical | Insulin % of theoretical | 4-CNAB % of theoretical |
| 0 minutes | 58.9 | 98.6 | 92.9 | 66.9 | 100.2 | 103.9 |
| 2 minutes | 26.9 | 98.9 | - | 55.2 | 102.1 | 105.4 |
| 4 minutes | 19.3 | 99.0 | - | 45.1 | 106.3 | 111.5 |
| 6 minutes | 5.4 | 98.8 | - | 34.5 | 103.1 | 106.0 |
| 8 minutes | 3.1 | 98.6 | - | 24.5 | 101.7 | 101.8 |
| 10 minutes | 2.5 | 98.2 | 92.5 | 15.1 | 101.3 | 103.4 |
| 15 minutes | | | 92.7 | | 100.9 | 104.0 |
| 30 minutes | | | 94.7 | | 100.6 | 104.0 |
| 45 minutes | | | - | | 99.1 | 102.3 |
| 1 hour | | | 92.7 | | 95.9 | 100.0 |
| 2 hours | | | 94.6 | | - | - |
| 24 hours | | | 100.4 | | 105.6 | 111.3 |

These results show that insulin is stable in SIF without pancreatin and degrades in presence of the enzyme. Insulin is more stable in SIF with and without enzyme than in SGF with and without enzyme. At the first sampling time point (0 minuts) only 3.0% insulin remained in SGF with enzymes while 58.9% and 66.9% insulin remained in SIF.

### Example 5

### Effect of Formulation on Insulin Absorption and Action

Six formulations containing insulin shown in Table 15 were prepared as follows.

**Table 15**

| ID | Formulation |
|---|---|
| 1 | Fast Disintegrating Tablets - 1. Insulin, 4-CNAB, 0.4% w/w povidone, 10% w/w Polyplasdone XL, 50.7% w/w Emcocel HD90, 1% w/w SLS and 1% w/w Magnesium Stearate |
| 2 | Fast Disintegrating Tablets - 2. Insulin, 4-CNAB, 0.4% w/w povidone 10% w/w Polyplasdone XL, 50.2% w/w Prosolv HD90, 1% w/w SLS and 1% w/w Magnesium Stearate |
| 3 | Tablets with Emcompress. Insulin, 4-CNAB, 0.4% w/w povidone, ∼29.1% w/w Emcompress, 1% w/w SLS and 1 % w/w Magnesium Stearate |
| 4 | Tablets with Mg Stearate only. Insulin, 4-CNAB, 0.4% w/w povidone, and 1 % w/w Magnesium Stearate |
| 5 | Tablets with Anhydrous Emcompress. Insulin, 4-CNAB, 0.4% w/w povidone, ∼29.1% w/w Anhydrous Emcompress, 1% w/w SLS and 1% w/w Magnesium Stearate |
| 6 | Co-dried capsules. Insulin and 4-CNAB were dissolved in water. The solution was co-dried and the resulting powder was filled into hard gelatin capsules. |

Polyplasdone XL, is available from International Specialty Products, Wilmington DE.; Emcocel HD90, Prosolv HD90, Emcompress and Anhydrous Emcompress is available from JRS Pharma, Patterson, NY.

The formulations were fed to rhesus monkeys in doses containing 100 mg/kg of 4-CNAB and 13 U/kg insulin. Groups of four rhesus monkeys, two males and two females, were fasted for at least 12 hrs prior to dosing and up to 4 hrs after dosing. Water was withheld approximately 1 hr before dosing and up to 2 hrs after dosing after which it was permitted *ad libitum.* The dosing was followed by a 5 ml water flush. Blood samples (approximately 2 ml each) were collected by venipuncture at 15 minutes before dosing and at 5, 10, 15, 20, 30, 45 minutes and 1, 1.5, 2, 3, 4 hr after dosing. Each blood sample was divided into two portions. One portion was allowed to clot at room temperature and centrifuged at 2-8°C for 10 minutes at 3000 rpm. The serum obtained was aliquoted into two portions and stored at -70° C until shipment. One sample was shipped to Emisphere on dry ice for insulin analysis by ELISA while the other was retained by the CRO for serum glucose analysis. The second portion of the blood was kept on wet ice for up to 30 minutes and centrifuged at 2-8°C for 10 minutes at 3000 rpm. The plasma obtained was shipped to Emisphere on dry ice for analysis of 4-CNAB content by HPLC. Each formulation was administered to 4 rhesus monkeys, except formulation 1, which was administered to 8 rhesus monkeys. Blood samples were taken at predetermined intervals as described above and assayed for insulin and glucose levels. The results are shown in table 16 and in Figures 17 and 18 .

**Table 16**

| Form.ID | Disint. Time | Onset of glucose lowering | Insulin tₘₐₓ | Serum Glucose tₘᵢₙ (Cmin, % change from baseline + SE) | Duration of Action (Glucose Level) |
|---|---|---|---|---|---|
| 1 | 35 sec | 4 min | 30 min | 120 min (-33.57 ± 8.25%) | 240 min+ (-21.87 ± 6.64%) |
| 2 | 23 sec | 8 min | 10 min | 60 min (-25.6 ± 13.91%) | 240 min+ (-26.17 ± 7.39%) |
| 3 | 6 min 43 sec | 7 min | 60 min | 60 min (-50.68 ± 4.41%) | 240 min+ (-33.96 ± 3.36%) |
| 4 | 8 min 40 sec | 2 min | 15 min | 45 min (-46.27 ± 7.78%) | 240 min (-0.80 ± 13.98%) |
| 5 | 7 min 6 sec | 6 min | 20 min | 60 min (-58.13 ± 3.89%) | 240 min+ (-10.14 ± 0%) |
| 6 | | 9 min | 15 min | 45 min (-26.96 ± 13.13%) | 90 min (18.43 ± 23.84%) |

Disintegration time was determined in water at 37 ± 2°C using the method described in USP <701>. Multiple tubes containing water are placed in a basket-rack assembly immersed in a water bath maintained at 37 ± 2°C. The basket-rack assembly raises and lowers the tubes at a constant frequency. The tablets are placed in the tubes and are periodically examined to determine if they have disintegrated completely. Each tablet is tested in six different tubes. If 1 or 2 tablets fails to consistently disintegrate, the procedure is repeated on additional tablets. The average maximum concentration of insulin (Cₘₐₓ) was determined for each group based upon the serum levels of insulin measured as described above. If the blood glucose levels in the primates falls to very low levels (< 1 mmol/L) during the experiment they are administered dextrose in order to bring the blood glucose up to a safe level. The average Cₘₐₓ for each group, as well as the number of rhesus monkeys rescued, is shown in table 17.

**Table 17**

| Formulation ID | Insulin Cₘₐₓ (*µ*U/ml) | No. of primates dosed | No. of primates rescued |
|---|---|---|---|
| 1 | 0.6585 ± 0.6585 | 8 | 0 |
| 2 | 3.99 ± 3.99 | 4 | 0 |
| 3 | 70.81 ± 43.22 | 4 | 2 |
| 4 | 51.65 ± 30.69 | 4 | 1 |
| 5 | 60.46 ± 34.56 | 4 | 3 |
| 6 | 31.75 ± 31.54 | 4 | 1 |

### Example 6

### Preparation of Enteric Coated Tablets

Capsules were manufactured by encapsulating 300 mg of a formulation including 150 units insulin, 200 mg 4-CNAB, 0.4% w/w povidone, ∼29.1% w/w Emcompress, 1% w/w SLS, and 1% w/w magnesium stearate into size 2 white opaque capsules. The capsules were first coated with a subcoat consisting of Opadry clear for a weight gain of 5 % followed by an enteric coat of 20 % weight gain for a total weight gain on the capsules of 25 %.

Tablets were manufactured by pressing 300 mg of the formulation described above into tablets. An 10% weight gain enteric coat was applied. The formulations for the subcoats and enteric coats are shown in table 18 below.

**Table 18**

| | **Tablets** | **Capsules** |
|---|---|---|
| Ingredients | % w/w | % w/w |
| **SUBCOAT** | | |
| Opadry Clear | NA | 8.0 |
| Milli Q Water | NA | 92.0 |
| Total | NA | 100.0 |
| **ENTERIC COAT** | | |
| Eudragit L30D55 | 49.4 | 49.4 |
| Talc | 3.7 | 3.7 |
| Triethyl Citrate | 1.5 | 1.5 |
| Milli Q Water | 45.4 | 45.4 |
| Total | 100.0 | 100.0 |

Opadry™ Clear is available from Colorcon, of West Point, PA.

Milli Q Water is highly purified water and is available from Millipore of Billerica, Massachusetts.

Eudragit L30D55 is available from Degussa AG, Parsippany, NJ.

To verify the effectiveness of the enteric coat, the coated capsules and tablets were placed in 0.1 N HCl for two hours or pH 6.8 phosphate buffer for one hour. The coated capsules and tablets did not dissolve in the 0.1 N HCl, but did dissolve in the pH 6.8 phosphate buffer.

### Example 7

### SNAC Micro Beads Coated With Heparin

5 g of SNAC and 0.5 g of magnesium stearate were mixed. 0.02 g of the mixed powder was fed into a die. Small beads of SNAC and magnesium stearate were made at 1200 PSI bar pressure The beads had a round/ball shape size of about 0.2 mm to about 2.0 mm. The SNAC beads were then coated with 2.5 g of heparin, in liquid form, by a rotary method and dried under vacuum oven at 40° C for 10 hours.

### Example 8

### Micronized SNAP with Heparin

SNAD was screened through a 35 mesh Tyler standard sieve. The SNAD was milled with a Glen Mills, Model S100 centrifugal ball mill (Clifton, NJ) equipped with a 250 mL stainless steel grinding jar and 30 mm (440c) diameter stainless steel balls was used. The process parameters investigated were (1) number of balls used, (2) duration of milling, (3) milling speed, and (4) milling jar total charge. A Malvern Mastersizer 2000 equipped with a Scirocco 2000 dry accessory was used for particle size determination. A Kratos XRD 6000 (version 4.1) X-ray powder diffractometer scanning over the 2θ range 5-40° 2θ was used for monitoring crystallinity changes. The diverging, scattering, and receiving slits were 1°, 1°, and 0.3 mm respectively. A Brinkmann 737 KF coulometer was used for moisture content determination while a Quantachrome Nova 3000 Series Surface Area Analyzer was used for specific surface area determination.

The results indicated that the particle size distribution of pre-screened SNAD was d(0.1) = 1.6 µm, d(0.5) = 10.5 µm, and d(0.9) = 314.9 µm. The data obtained using different numbers of balls ranging from 1 to 5 indicated that the optimum number of balls for the charge used was 2. The use of 2 balls yielded the particle size d(0.1) = 1.1 µm, d(0.5) = 12.0 µm, and d(0.9) = 154.3 µm.

An evaluation of the effect of milling time for a fixed number of balls and charge indicated that a milling time of 120 minutes was optimum resulting in the particle size distribution, d(0.1) = 2.0 µm, d(0.5) = 15.4, and d(0.9) = 62.9 µm.

An evaluation of the milling speeds 100, 300, and 500 rpm indicated that optimum milling was obtained at 300 rpm. This speed yielded the particle size distribution, d(0.9) = 62.9 µm compared to unmilled SNAD d(0.9) = 314.9 µm.

A charge of 37 mL of the 250 mL milling jar provided better milling compared to 75 and 112 mL. The powder X-ray diffraction analysis indicated that milling did not result in crystallinity changes for SNAD. The Karl Fischer moisture content determination indicated no significant changes in moisture content.

The SNAD was then mixed with heparin.

### Example 9

### Micronized SNAC with Micronized Heparin

SNAC and heparin were micronized separately by the procedure described in Example 8 with 2 balls at 200 rpm for 120 minutes and then mixed together. The micronized SNAC had a d(0.5) of 7.574 µm SNAC/heparin capsules having the formulations shown in table 19 below were prepared by hand packing them into hard gelatin capsules.

**Table 19**

| Ingredient | Formulation (mg/capsule) | |
|---|---|---|
| | A | B |
| Micronized SNAC | 125 | 125 |
| Micronized Heparin USP (30,000 U) | 158 | 158 |
| Propylene Glycol Monocaprylate¹ | 105 | 105 |
| Sodium lauryl sulfate | 7 | 7 |
| PEG 300² | 305 | 270 |
| Water | - | 35 |
| Total | 700 | 700 |

| | | |
|---|---|---|
| ¹ - Propylene glycol monocaprylate is available as Capmul™ PG 8 from Abitec Corporation of Columbus, OH. ² - PEG 300 is available as Carbowax™ 300 from Dow Chemical Co. of Midland, MI. | | |

The heparin, SNAC, and sodium lauryl sulfate were mixed. Separately, the PEG 300, propylene glycol monocaprylate, and water (for formulation B) were mixed. 50% of the liquid PEG 300/propylene glycol monocaprylate mixture was transferred to a mortar. The heparin, SNAC, and sodium lauryl sulfate blended powder was added little by little and triturated with the liquid in the mortar and pestle. The capsules were then packed with the resulting mixture.

### Example 10

### Micronized SNAC/Heparin

Heparin (118.5 mg/dose (22,500 rpm)) and SNAC (125 mg/dose) were dry mixed, screened through a 35 mesh screen, and milled for about 4 minutes with a ball mill. The mixture was packed into capsules (Capsugel Size 1 capsules (Greenwood, SC)).

The capsules were administered to rhesus monkeys (2 capsules per monkey) by the following procedure. Rhesus monkeys weighing between 3.5 - 5.0 kg were fasted overnight before the experiments and food was returned about 2 hours after dosing. Water was withheld from 30 minutes prior to dosing until 30 minutes after dosing, except for those quantities used for dosing. Each dosage form was delivered to the rear of the mouth using a pill gun. After release of the dosage form, 5 ml of reverse osmosis water was administered into the oral cavity to facilitate swallowing. Following delivery, the oral cavity was inspected to ensure that the capsule was swallowed. Antifactor Xa from blood samples was measured over 6 hours.

The results are shown in Figure 19.

### Example 11

### Micronized SNAC/Heparin

Capsules containing micronized SNAC/heparin as shown in table 20 below were prepared as follows.

A solution of heparin and SNAC was prepared as follows. The required amounts of heparin and SNAC were weighed out and water, which was previously adjusted to a pH of about 8 with sodium hydroxide, was added. The pH of the resulting solution was in the range of about 7.3 - 7.5. The solution pH was adjusted to a pH of about 8 with sodium hydroxide. The solution was then dried in a RotoVap apparatus at 50° C under vacuum. The evaporating was done using the program outlined below.
1. Immediate reduction of vacuum from 101 kPa to 26.7 kPa (760 torr to 200 torr)
2. Reduction of vacuum pressure from 26.7 to 13.3 kPa (200 to 100 torr) in 2 minutes
3. Reduction of vacuum pressure from 13.3 to 6.7 kPa (100 to 50 torr) in 2 minutes
4. Reduction of vacuum pressure from 6.7 to 3.3 kPa (50 to 25 torr) in 4 minutes
5. Reduction of vacuum pressure from 3.3 to 2.0 kPa (25 to 15 torr) in 4 minutes
6. Reduction of vacuum pressure from 2.0 to 1.3 kPa (15 to 10 torr) in 2 minutes
7. Evaporating at 10 ± 2 torr and 70 rpm in 30 minutes
8. Switch to 50 rpm manually and continue with evaporating for 4 hours

The sample was vacuum dried overnight. The resulting powder was then micronized and filled into capsules to give the desired dose.

**Table 20**

| Ingredient | Formulation (mg/capsule) | | |
|---|---|---|---|
| | A | B | C |
| Micronized SNAC | 198 | 173 | 210.6 |
| Micronized Heparin USP | 72 (13104 USP heparin units) | 63 (11466 USP heparin units) | 78 (14196 USP heparin units) |
| Total | 270 | 236 | 288.6 |

## Claims

1. Particles comprising a delivery agent and a biologically active agent, wherein the particles have a median particle size of 250 to 425 micrometers and the delivery agent is a compound of Formula A, or a pharmaceutically acceptable salt thereof, 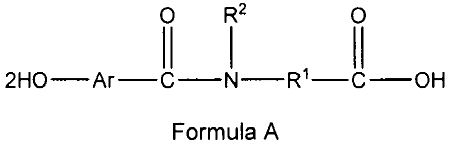 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁C₁₀ alkyl), phenyKC₁C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

2. A pharmaceutical formulation comprising particles having a median particle size of 250 to 425 micrometers, the particles comprising a delivery agent and a biologically active agent,
wherein the delivery agent is a compound of Formula A, or a pharmaceutically acceptable salt thereof, 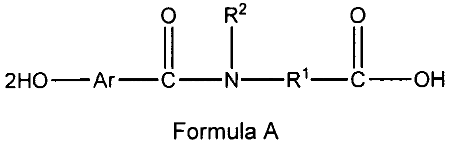 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

3. A pharmaceutical formulation comprising a delivery agent and a biologically active agent, wherein the delivery agent is in the form of particles having a median particle size of 250 to 425 micrometers, and the delivery agent is a compound of Formula A, or a pharmaceutically acceptable salt thereof, 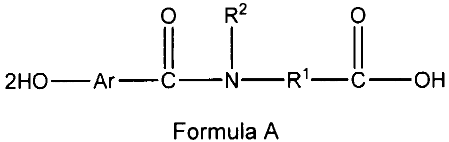 wherein
Ar is phenyl or naphthyl;
Ar is optionally substituted with one or more of -OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

4. The pharmaceutical formulation of claim 3, wherein the delivery agent particles are compressed to form micro-beads.

5. The pharmaceutical formulation of claim 4, wherein the micro-beads are coated with an active agent.

6. The pharmaceutical formulation of claim 5, wherein the active agent is insulin or heparin.

7. The pharmaceutical formulation of any of claims 4-6, wherein the micro-beads have a diameter of 0.2 mm to 2.0 mm.

8. A pharmaceutical formulation comprising a delivery agent and a biologically active agent, wherein the biologically active agent is in the form of particles having a median particle size of 250 to 425 micrometers, and the delivery agent is a compound of Formula A, or a pharmaceutically acceptable salt thereof, 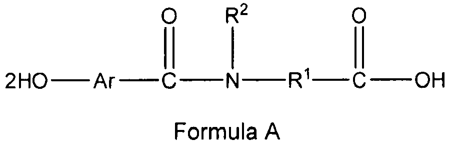 wherein
Ar is phenyl or naphthyl;
Ar is optionally substituted with one or more of-OH, halogen, C₁-C₄ alkyl, C₁-C₄ alkenyl, C₁-C₄ alkoxy or C₁-C₄ haloalkoxy;
R¹ is C₃-C₂₀ alkyl, C₄-C₂₀ alkenyl, phenyl, naphthyl, (C₁-C₁₀ alkyl) phenyl, (C₁-C₁₀ alkenyl)phenyl, (C₁-C₁₀ alkyl) naphthyl, (C₁-C₁₀ alkenyl) naphthyl, phenyl(C₁-C₁₀ alkyl), phenyl(C₁-C₁₀ alkenyl), naphthyl(C₁-C₁₀ alkyl), or naphthyl(C₁-C₁₀ alkenyl);
R¹ is optionally substituted with C₁-C₄ alkyl, C₂-C₄ alkenyl, C₁-C₄ alkoxy, C₁-C₄ haloalkoxy, -OH, -SH, -CO₂R⁹, or any combination thereof;
R² is hydrogen, C₁-C₄ alkyl, or C₂-C₄ alkenyl; and
R¹ is optionally interrupted by oxygen, nitrogen, sulfur or any combination thereof.

9. The pharmaceutical formulation of any one of claims 2 to 8, wherein the delivery agent compound is selected from N-(8-[2-hydroxybenzoyl]-amino)caprylic acid, N- (10-[2-hydroxybenzoyl]-amino)decanoic acid, 8-(2-hydroxy-4- methoxybenzoylamino)octanoic acid, 8-(2-hydroxy-5-chlorobenzoylamino)-octanoic acid, 4-[(2-hydroxy-4-chlorobenzoyl)-amino]butanoic acid, and pharmaceutically acceptable salts thereof.

10. The pharmaceutical formulation of any one of claims 2 to 8, wherein the delivery agent compound is N-(8-[2-hydroxybenzoyl]-ammo)caprylic acid or a pharmaceutically acceptable salt thereof.

11. The pharmaceutical formulation of any one of claims 2 to 8, wherein the delivery agent compound is N-(10-[2-hydroxybenzoyl]-amino)decanoic acid or a pharmaceutically acceptable salt thereof.

12. The pharmaceutical formulation of any one of claims 2 to 8, wherein the delivery agent compound is 4-[(2-hydroxy-4-chloro-benzoyl)-amino]butanoic acid or a pharmaceutically acceptable salt thereof.

13. The pharmaceutical formulation of any one of claims 2 to 8, wherein the biologically active agent is selected from proteins, polypeptides, peptides, hormones, and polysaccharides.

14. The pharmaceutical formulation of any one of claims 2 to 8, wherein the biologically active agent is selected from the following, including synthetic, natural or recombinant sources thereof: growth hormones; growth hormone releasing hormones; growth hormone releasing factor, interferons; interleukin-1; interleukin-2; insulin, optionally having counter ions including zinc, sodium, calcium and ammonium; insulin-like growth factor; heparin; calcitonin; erythropoietin; atrial naturetic factor; antigens; monoclonal antibodies; somatostatin; protease inhibitors; adrenocorticotropin, gonadotropin releasing hormone; oxytocin; leutinizing-hormone-releasing-hormone; follicle stimulating hormone; glucocerebrosidase; thrombopoietin; filgrastim; prostaglandins; cyclosporin; vasopressin; cromolyn sodium; vancomycin; desferrioxamine; bisphosphonates; parathyroid hormone; anti-migraine agents; glucagon-like peptide 1 (GLP-I); antimicrobials; vitamins; analogs, fragments, mimetics or polyethylene glycol (PEG)-modified derivatives of these compounds; or any combination thereof.

15. The pharmaceutical formulation of any one of claims 2 to 8, wherein the biologically active agent is insulin.

16. The pharmaceutical formulation of any one of claims 2 to 8, wherein the biologically active agent is heparin.

17. The pharmaceutical formulation of any one of claims 2 to 8, wherein the biologically active agent is unfractionated heparin.

18. The pharmaceutical formulation of any one of claims 2 to 8, wherein the biologically active agent is low molecular weight heparin.

19. The pharmaceutical formulation of any one of claims 2 to 18, wherein the particles have an enteric coating.

20. A solid dosage unit form comprising the pharmaceutical formulation of any one of claims 2 to 19.

21. The solid dosage unit form of claim 20, further comprising a disintegrant.

22. The solid dosage unit form of claim 21, wherein the disintegrant is a super disintegrant.

23. The solid dosage unit form of claim 22, wherein the super disintegrant is sodium starch glycolate or croscarmellose sodium.

24. The solid dosage unit form of claim 22, wherein the super disintegrant is an extra particle super disintegrant.

25. The solid dosage unit form of any of claims 20-24, wherein the solid dosage unit form is in the form of a tablet.

26. The solid dosage unit form of any of claims 20-24, wherein the solid dosage unit form is in the form of a capsule.

27. A pharmaceutical formulation according to any one of claims 2 to 19 for use in treating diabetes in a mammal in need thereof.

28. The formulation for use according to claim 27, wherein the delivery agent compound is 4-[(2-hydroxy-4-chloro-benzoyl)-amino]butanoic acid or a pharmaceutically acceptable salt thereof.

29. A pharmaceutical formulation according to any one of claims 2 to 19 for use in treating impaired glucose tolerance, early stage diabetes, or late stage diabetes or achieving glucose homeostasis in a human in need thereof.

30. The formulation for use according to claim 29, comprising administering the pharmaceutical formulation on a chronic basis.

31. A pharmaceutical formulation according to any one of claims 2 to 19 for use in treating a human diabetic patient, comprising orally administering the pharmaceutical formulation on a chronic basis.

## Patentansprüche

1. Teilchen, die ein Verabreichungsmittel und ein biologisch aktives Mittel umfassen, wobei die Teilchen eine mittlere Teilchengröße von 250 bis 425 µm aufweisen und das Verabreichungsmittel eine Verbindung der Formel A oder ein pharmazeutisch verträgliches Salz davon ist, 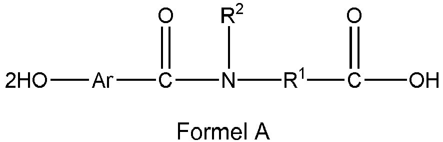 wobei
Ar Phenyl oder Naphthyl ist;
Ar optional mit einem oder mehreren aus -OH, Halogen, C₁-C₄-Alkyl, C₁-C₄-Alkenyl, C₁-C₄-Alkoxy oder C₁-C₄-Haloalkoxy substituiert ist;
R¹ C₃-C₂₀-Alkyl, C₄-C₂₀-Alkenyl, Phenyl, Naphthyl, (C₁-C₁₀-Alkyl)phenyl, (C₁-C₁₀-Alkenyl)phenyl, (C₁-C₁₀-Alkyl)naphthyl, (C₁-C₁₀-Alkenyl)naphthyl, Phenyl(C₁-C₁₀-alkyl), Phenyl(C₁-C₁₀-alkenyl), Naphthyl(C₁-C₁₀-alkyl) oder Naphthyl(C₁-C₁₀-alkenyl) ist;
R¹ optional mit C₁-C₄-Alkyl, C₂-C₄-Alkenyl, C₁-C₄-Alkoxy, C₁-C₄-Haloalkoxy, -OH, -SH, -CO₂R⁹ oder einer Kombination derselben substituiert ist;
R² Wasserstoff, C₁-C₄-Alkyl oder C₂-C₄-Alkenyl ist; and
R¹ optional durch Sauerstoff, Stickstoff, Schwefel oder eine Kombination derselben unterbrochen ist.

2. Pharmazeutische Formulierung, umfassend Teilchen mit einer mittleren Teilchengröße von 250 bis 425 µm, wobei die Teilchen ein Verabreichungsmittel und ein biologisch aktives Mittel umfassen,
wobei das Verabreichungsmittel eine Verbindung der Formel A oder ein pharmazeutisch verträgliches Salz davon ist, 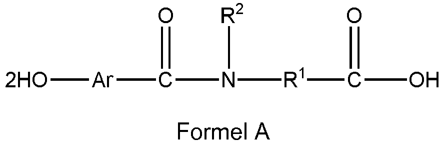 wobei
Ar Phenyl oder Naphthyl ist;
Ar optional mit einem oder mehreren aus -OH, Halogen, C₁-C₄-Alkyl, C₁-C₄-Alkenyl, C₁-C₄-Alkoxy oder C₁-C₄-Haloalkoxy substituiert ist;
R¹ C₃-C₂₀-Alkyl, C₄-C₂₀-Alkenyl, Phenyl, Naphthyl, (C₁-C₁₀-Alkyl)phenyl, (C₁-C₁₀-Alkenyl)phenyl, (C₁-C₁₀-Alkyl)naphthyl, (C₁-C₁₀-Alkenyl)naphthyl, Phenyl(C₁-C₁₀-alkyl), Phenyl(C₁-C₁₀-alkenyl), Naphthyl(C₁-C₁₀-alkyl) oder Naphthyl(C₁-C₁₀-alkenyl) ist;
R¹ optional mit C₁-C₄-Alkyl, C₂-C₄-Alkenyl, C₁-C₄-Alkoxy, C₁-C₄-Haloalkoxy, -OH, -SH, -CO₂R⁹ oder einer Kombination derselben substituiert ist;
R² Wasserstoff, C₁-C₄-Alkyl oder C₂-C₄-Alkenyl ist; and
R¹ optional durch Sauerstoff, Stickstoff, Schwefel oder eine Kombination derselben unterbrochen ist.

3. Pharmazeutische Formulierung, umfassend ein Verabreichungsmittel und ein biologisch aktives Mittel, wobei das Verabreichungsmittel in Form von Teilchen mit einer mittleren Teilchengröße von 250 bis 425 µm vorliegt und das Verabreichungsmittel eine Verbindung der Formel A oder ein pharmazeutisch verträgliches Salz davon ist, 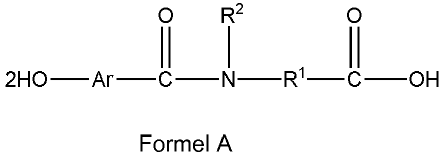 wobei
Ar Phenyl oder Naphthyl ist;
Ar optional mit einem oder mehreren aus -OH, Halogen, C₁-C₄-Alkyl, C₁-C₄-Alkenyl, C₁-C₄-Alkoxy oder C₁-C₄-Haloalkoxy substituiert ist;
R¹ C₃-C₂₀-Alkyl, C₄-C₂₀-Alkenyl, Phenyl, Naphthyl, (C₁-C₁₀-Alkyl)phenyl, (C₁-C₁₀-Alkenyl)phenyl, (C₁-C₁₀-Alkyl)naphthyl, (C₁-C₁₀-Alkenyl)naphthyl, Phenyl(C₁-C₁₀-alkyl), Phenyl(C₁-C₁₀-alkenyl), Naphthyl(C₁-C₁₀-alkyl) oder Naphthyl(C₁-C₁₀-alkenyl) ist;
R¹ optional mit C₁-C₄-Alkyl, C₂-C₄-Alkenyl, C₁-C₄-Alkoxy, C₁-C₄-Haloalkoxy, -OH, -SH, -CO₂R⁹ oder einer Kombination derselben substituiert ist;
R² Wasserstoff, C₁-C₄-Alkyl oder C₂-C₄-Alkenyl ist; and
R¹ optional durch Sauerstoff, Stickstoff, Schwefel oder eine Kombination derselben unterbrochen ist.

4. Pharmazeutische Formulierung nach Anspruch 3, wobei die Verabreichungsmittelteilchen unter Bildung von Mikrokügelchen komprimiert sind.

5. Pharmazeutische Formulierung nach Anspruch 4, wobei die Mikrokügelchen mit einem Wirkstoff überzogen sind.

6. Pharmazeutische Formulierung nach Anspruch 5, wobei der Wirkstoff Insulin oder Heparin ist.

7. Pharmazeutische Formulierung nach einem der Ansprüche 4 bis 6, wobei die Mikrokügelchen einen Durchmesser von 0,2 mm bis 2,0 mm aufweisen.

8. Pharmazeutische Formulierung, umfassend ein Verabreichungsmittel und ein biologisch aktives Mittel, wobei das biologisch aktive Mittel in Form von Teilchen mit einer mittleren Teilchengröße von 250 bis 425 µm vorliegt und das Verabreichungsmittel eine Verbindung der Formel A oder ein pharmazeutisch verträgliches Mittel Salz davon ist, 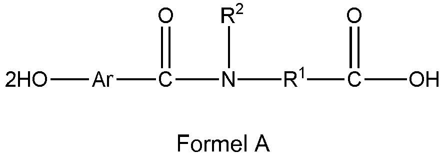 wobei
Ar Phenyl oder Naphthyl ist;
Ar optional mit einem oder mehreren aus -OH, Halogen, C₁-C₄-Alkyl, C₁-C₄-Alkenyl, C₁-C₄-Alkoxy oder C₁-C₄-Haloalkoxy substituiert ist;
R¹ C₃-C₂₀-Alkyl, C₄-C₂₀-Alkenyl, Phenyl, Naphthyl, (C₁-C₁₀-Alkyl)phenyl, (C₁-C₁₀-Alkenyl)phenyl, (C₁-C₁₀-Alkyl)naphthyl, (C₁-C₁₀-Alkenyl)naphthyl, Phenyl(C₁-C₁₀-alkyl), Phenyl(C₁-C₁₀-alkenyl), Naphthyl(C₁-C₁₀-alkyl) oder Naphthyl(C₁-C₁₀-alkenyl) ist;
R¹ optional mit C₁-C₄-Alkyl, C₂-C₄-Alkenyl, C₁-C₄-Alkoxy, C₁-C₄-Haloalkoxy, -OH, -SH, -CO₂R⁹ oder einer Kombination derselben substituiert ist;
R² Wasserstoff, C₁-C₄-Alkyl oder C₂-C₄-Alkenyl ist; and
R¹ optional durch Sauerstoff, Stickstoff, Schwefel oder eine Kombination derselben unterbrochen ist.

9. Pharmazeutische Formulierung nach einem der Ansprüche 2 bis 8, wobei die Verabreichungsmittelverbindung ausgewählt ist aus N-(8-[2-Hydroxybenzoyl]amino)caprylsäure, N-(10-[2-Hydroxybenzoyl]amino)decansäure, 8-(2-Hydroxy-4-methoxybenzoylamino)octansäure, 8-(2-Hydroxy-5-chlorbenzoylamino)octansäure, 4-[(2-Hydroxy-4-chlorbenzoyl)amino]butansäure, und pharmazeutisch verträglichen Salzen davon.

10. Pharmazeutische Formulierung nach einem der Ansprüche 2 bis 8, wobei die Verabreichungsmittelverbindung N-(8-[2-Hydroxybenzoyl]amino)caprylsäure oder ein pharmazeutisch verträgliches Salz davon ist.

11. Pharmazeutische Formulierung nach einem der Ansprüche 2 bis 8, wobei die Verabreichungsmittelverbindung N-(10-[2-Hydroxybenzoyl]amino)decansäure oder ein pharmazeutisch verträgliches Salz davon ist.

12. Pharmazeutische Formulierung nach einem der Ansprüche 2 bis 8, wobei die Verabreichungsmittelverbindung 4-[(2-Hydroxy-4-chlorbenzoyl)amino]butansäure oder ein pharmazeutisch verträgliches Salz davon ist.

13. Pharmazeutische Formulierung nach einem der Ansprüche 2 bis 8, wobei das biologisch aktive Mittel ausgewählt ist aus Proteinen, Polypeptiden, Peptiden, Hormonen und Polysacchariden.

14. Pharmazeutische Formulierung nach einem der Ansprüche 2 bis 8, wobei das biologisch aktive Mittel ausgewählt ist aus den folgenden, einschließlich synthetischen, natürlichen oder rekombinanten Quellen davon: Wachstumshormonen; Wachstumshormon freisetzenden Hormonen; Wachstumshormon freisetzendem Faktor, Interferonen; Interleukin-1; Interleukin-2; Insulin, gegebenenfalls mit Gegenionen einschließlich Zink, Natrium, Calcium und Ammonium; insulinähnlichem Wachstumsfaktor; Heparin; Calcitonin; Erythropoietin; atrialem natriuretischem Faktor; Antigenen; monoklonalen Antikörpern; Somatostatin; Proteaseinhibitoren; Adrenocorticotropin, Gonadotropin-Releasing-Hormon; Oxytocin; luteinisierendem Hormon-Releasing-Hormon; follikelstimulierendem Hormon; Glucocerebrosidase; Thrombopoietin; Filgrastim; Prostaglandinen; Cyclosporin; Vasopressin; Cromolyn-Natrium; Vancomycin; Desferrioxamin; Bisphosphonaten; Parathormon; Mitteln gegen Migräne; Glucagon-ähnlichem Peptid 1 (GLP-I); antimikrobiellen Stoffen; Vitaminen; Analoga, Fragmenten, Mimetika oder Polyethylenglykol (PEG)-modifizierten Derivaten dieser Verbindungen; oder irgendeiner Kombination davon.

15. Pharmazeutische Formulierung nach einem der Ansprüche 2 bis 8, wobei das biologisch aktive Mittel Insulin ist.

16. Pharmazeutische Formulierung nach einem der Ansprüche 2 bis 8, wobei das biologisch aktive Mittel Heparin ist.

17. Pharmazeutische Formulierung nach einem der Ansprüche 2 bis 8, wobei das biologisch aktive Mittel unfraktioniertes Heparin ist.

18. Pharmazeutische Formulierung nach einem der Ansprüche 2 bis 8, wobei das biologisch aktive Mittel Heparin mit niedrigem Molekulargewicht ist.

19. Pharmazeutische Formulierung nach einem der Ansprüche 2 bis 18, wobei die Teilchen eine magensaftresistente Beschichtung aufweisen.

20. Feste Dosierungseinheitsform, umfassend die pharmazeutische Formulierung nach einem der Ansprüche 2 bis 19.

21. Feste Dosierungseinheitsform nach Anspruch 20, ferner umfassend ein Sprengmittel.

22. Feste Dosierungseinheitsform nach Anspruch 21, wobei das Sprengmittel ein Supersprengmittel ist.

23. Feste Dosierungseinheitsform nach Anspruch 22, wobei das Supersprengmittel Natriumstärkeglycolat oder Croscarmellose-Natrium ist.

24. Feste Dosierungseinheitsform nach Anspruch 22, wobei das Supersprengmittel ein Extrateilchen-Supersprengmittel ist.

25. Feste Dosierungseinheitsform nach einem der Ansprüche 20-24, wobei die feste Dosierungseinheitsform in Form einer Tablette vorliegt.

26. Feste Dosierungseinheitsform nach einem der Ansprüche 20-24, wobei die feste Dosierungseinheitsform in Form einer Kapsel vorliegt.

27. Pharmazeutische Formulierung nach einem der Ansprüche 2 bis 19 zur Verwendung bei der Behandlung von Diabetes bei einem Säugetier, das dies benötigt.

28. Formulierung zur Verwendung nach Anspruch 27, wobei die Verabreichungsmittelverbindung 4-[(2-Hydroxy-4-chlorbenzoyl)amino]butansäure oder ein pharmazeutisch verträgliches Salz davon ist.

29. Pharmazeutische Formulierung nach einem der Ansprüche 2 bis 19 zur Verwendung bei der Behandlung von gestörter Glukosetoleranz, Diabetes im Frühstadium, Diabetes im Spätstadium oder zur Erzielung einer Glukosehomöostase bei einem Menschen, der dies benötigt.

30. Formulierung zur Verwendung nach Anspruch 29, umfassend die chronische Verabreichung der pharmazeutischen Formulierung.

31. Pharmazeutische Formulierung nach einem der Ansprüche 2 bis 19 zur Verwendung bei der Behandlung eines menschlichen diabetischen Patienten, umfassend die chronische orale Verabreichung der pharmazeutischen Formulierung.

## Revendications

1. Particules comprenant un agent de libération et un agent biologiquement actif, les particules ayant une taille moyenne de particule de 250 à 425 micromètres et l'agent de libération étant un composé de Formule A, ou son sel pharmaceutiquement acceptable, 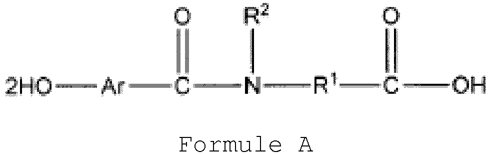 où
Ar est un groupe phényle ou naphtyle ;
Ar est éventuellement substitué par un ou plusieurs groupes parmi -OH, halogéno, alkyle en C₁ à C₄, alcényle en C₁ à C₄, alcoxy en C₁ à C₄ ou halogénoalcoxy en C₁ à C₄ ;
R¹ est un groupe alkyle en C₃ à C₂₀, alcényle en C₄ à C₂₀, phényle, naphtyle, (alkyl en C₁ à C₁₀) phényle, (alcényl en C₁ à C₁₀)phényle, (alkyl en C₁ à C₁₀) naphtyle, (alcényl en C₁ à C₁₀) naphtyle, phényl (alkyle en C₁ à C₁₀), phényl (alcényle en C₁ à C₁₀), naphtyl (alkyle en C₁ à C₁₀), ou naphtyl (alcényle en C₁ à C₁₀) ;
R¹ est éventuellement substitué par un groupe alkyle en C₁ à C₄, alcényle en C₂ à C₄, alcoxy en C₁ à C₄, halogénoalcoxy en C₁ à C₄, -OH, -SH, -CO₂R⁹, ou n'importe quelle combinaison de ceux-ci ;
R² est un groupe hydrogéno, alkyle en C₁ à C₄, ou alcényle en C₂ à C₄ ; et
R¹ est éventuellement interrompu par un atome d'oxygène, d'azote, de soufre ou n'importe quelle combinaison de ceux-ci.

2. Formulation pharmaceutique comprenant des particules ayant une taille moyenne de particule de 250 à 425 micromètres, les particules comprenant un agent de libération et un agent biologiquement actif,
l'agent de libération étant un composé de Formule A, ou son sel pharmaceutiquement acceptable, 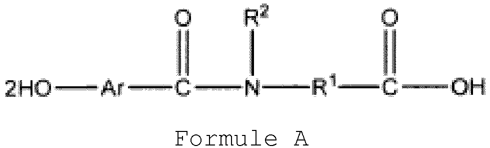 où
Ar est un groupe phényle ou naphtyle ;
Ar est éventuellement substitué par un ou plusieurs groupes parmi -OH, halogéno, alkyle en C₁ à C₄, alcényle en C₁ à C₄, alcoxy en C₁ à C₄ ou halogénoalcoxy en C₁ à C₄ ;
R¹ est un groupe alkyle en C₃ à C₂₀, alcényle en C₄ à C₂₀, phényle, naphtyle, (alkyl en C₁ à C₁₀) phényle, (alcényl en C₁ à C₁₀)phényle, (alkyl en C₁ à C₁₀) naphtyle, (alcényl en C₁ à C₁₀) naphtyle, phényl (alkyle en C₁ à C₁₀), phényl (alcényle en C₁ à C₁₀), naphtyl (alkyle en C₁ à C₁₀), ou naphtyl (alcényle en C₁ à C₁₀) ;
R¹ est éventuellement substitué par un groupe alkyle en C₁ à C₄, alcényle en C₂ à C₄, alcoxy en C₁ à C₄, halogénoalcoxy en C₁ à C₄, -OH, -SH, -CO₂R⁹, ou n'importe quelle combinaison de ceux-ci ;
R² est un groupe hydrogéno, alkyle en C₁ à C₄, ou alcényle en C₂ à C₄ ; et
R¹ est éventuellement interrompu par un atome d'oxygène, d'azote, de soufre ou n'importe quelle combinaison de ceux-ci.

3. Formulation pharmaceutique comprenant un agent de libération et un agent biologiquement actif, l'agent de libération se présentant sous la forme de particules ayant une taille moyenne de particule de 250 à 425 micromètres, et l'agent de libération étant un composé de Formule A, ou son sel pharmaceutiquement acceptable, 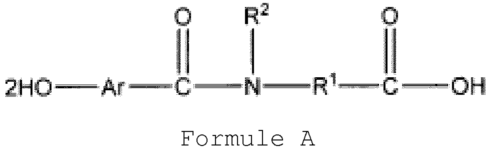 où
Ar est un groupe phényle ou naphtyle ;
Ar est éventuellement substitué par un ou plusieurs groupes parmi -OH, halogéno, alkyle en C₁ à C₄, alcényle en C₁ à C₄, alcoxy en C₁ à C₄ ou halogénoalcoxy en C₁ à C₄ ;
R¹ est un groupe alkyle en C₃ à C₂₀, alcényle en C₄ à C₂₀, phényle, naphtyle, (alkyl en C₁ à C₁₀) phényle, (alcényl en C₁ à C₁₀)phényle, (alkyl en C₁ à C₁₀) naphtyle, (alcényl en C₁ à C₁₀) naphtyle, phényl (alkyle en C₁ à C₁₀), phényl (alcényle en C₁ à C₁₀), naphtyl (alkyle en C₁ à C₁₀), ou naphtyl (alcényle en C₁ à C₁₀) ;
R¹ est éventuellement substitué par un groupe alkyle en C₁ à C₄, alcényle en C₂ à C₄, alcoxy en C₁ à C₄, halogénoalcoxy en C₁ à C₄, -OH, -SH, -CO₂R⁹, ou n'importe quelle combinaison de ceux-ci ;
R² est un groupe hydrogéno, alkyle en C₁ à C₄, ou alcényle en C₂ à C₄ ; et
R¹ est éventuellement interrompu par un atome d'oxygène, d'azote, de soufre ou n'importe quelle combinaison de ceux-ci.

4. Formulation pharmaceutique selon la revendication 3, les particules d'agent de libération étant comprimées pour former des microbilles.

5. Formulation pharmaceutique selon la revendication 4, les microbilles étant enrobées d'un agent actif.

6. Formulation pharmaceutique selon la revendication 5, l'agent actif étant l'insuline ou l'héparine.

7. Formulation pharmaceutique selon l'une quelconque des revendications 4 à 6, les microbilles ayant un diamètre de 0,2 mm à 2,0 mm.

8. Formulation pharmaceutique comprenant un agent de libération et un agent biologiquement actif, l'agent biologiquement actif se présentant sous la forme de particules ayant une taille moyenne de particule de 250 à 425 micromètres, et l'agent de libération étant un composé de Formule A, ou son sel pharmaceutiquement acceptable, 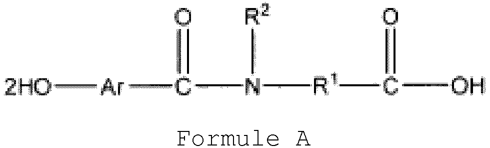 où
Ar est un groupe phényle ou naphtyle ;
Ar est éventuellement substitué par un ou plusieurs groupes parmi -OH, halogéno, alkyle en C₁ à C₄, alcényle en C₁ à C₄, alcoxy en C₁ à C₄ ou halogénoalcoxy en C₁ à C₄ ;
R¹ est un groupe alkyle en C₃ à C₂₀, alcényle en C₄ à C₂₀, phényle, naphtyle, (alkyl en C₁ à C₁₀) phényle, (alcényl en C₁ à C₁₀)phényle, (alkyl en C₁ à C₁₀) naphtyle, (alcényl en C₁ à C₁₀) naphtyle, phényl (alkyle en C₁ à C₁₀), phényl (alcényle en C₁ à C₁₀), naphtyl (alkyle en C₁ à C₁₀), ou naphtyl (alcényle en C₁ à C₁₀) ;
R¹ est éventuellement substitué par un groupe alkyle en C₁ à C₄, alcényle en C₂ à C₄, alcoxy en C₁ à C₄, halogénoalcoxy en C₁ à C₄, -OH, -SH, -CO₂R⁹, ou n'importe quelle combinaison de ceux-ci ;
R² est un groupe hydrogéno, alkyle en C₁ à C₄, ou alcényle en C₂ à C₄ ; et
R¹ est éventuellement interrompu par un atome d'oxygène, d'azote, de soufre ou n'importe quelle combinaison de ceux-ci.

9. Formulation pharmaceutique selon l'une quelconque des revendications 2 à 8, le composé agent de libération étant sélectionné parmi l'acide N-(8-[2-hydroxybenzoyl]-amino)caprylique, l'acide N-(10-[2-hydroxybenzoyl]-amino)décanoïque, l'acide 8-(2-hydroxy-4-méthoxybenzoylamino)octanoïque, l'acide 8-(2-hydroxy-5-chlorobenzoylamino)-octanoïque, l'acide 4-[(2-hydroxy-4-chlorobenzoyl)-amino]butanoique, et leurs sels pharmaceutiquement acceptables.

10. Formulation pharmaceutique selon l'une quelconque des revendications 2 à 8, le composé agent de libération étant l'acide N-(8-[2-hydroxybenzoyl]-amino)caprylique ou son sel pharmaceutiquement acceptable.

11. Formulation pharmaceutique selon l'une quelconque des revendications 2 à 8, le composé agent de libération étant l'acide N-(10-[2-hydroxybenzoyl]-amino)décanoïque ou son sel pharmaceutiquement acceptable.

12. Formulation pharmaceutique selon l'une quelconque des revendications 2 à 8, le composé agent de libération étant l'acide 4-[(2-hydroxy-4-chloro-benzoyl)-amino]butanoïque ou son sel pharmaceutiquement acceptable.

13. Formulation pharmaceutique selon l'une quelconque des revendications 2 à 8, l'agent biologiquement actif étant sélectionné parmi les protéines, les polypeptides, les peptides, les hormones, et les polysaccharides.

14. Formulation pharmaceutique selon l'une quelconque des revendications 2 à 8, l'agent biologiquement actif étant sélectionné parmi les suivants, comprenant leurs sources synthétiques, naturelles ou recombinantes : les hormones de croissance ; les hormones de libération d'hormone de croissance ; le facteur de libération d'hormone de croissance, les interférons ; l'interleukine-1 ; l'interleukine-2 ; l'insuline, ayant éventuellement des contre-ions comprenant le zinc, le sodium, le calcium et l'ammonium ; le facteur de croissance type insuline ; l'héparine ; la calcitonine ; l'érythropoïétine ; le facteur natriurétique auriculaire; les antigènes ; les anticorps monoclonaux ; la somatostatine ; les inhibiteurs de protéase ; l'adrénocorticotropine, l'hormone de libération de la gonadotropine ; l'oxytocine ; l'hormone de libération de l'hormone lutéinisante ; l'hormone de stimulation des follicules ; la glucocérébrosidase ; la thrombopoïétine ; le filgrastim ; les prostaglandines ; la cyclosporine ; la vasopressine ; le cromolyne sodique ; la vancomycine ; la desferrioxamine ; les bisphosphonates ; l'hormone parathyroïdienne ; les agents anti-migraineux ; le peptide type glucagon 1 (GLP-1) ; les antimicrobiens ; les vitamines ; les analogues, les fragments, les mimétiques ou les dérivés modifiés au polyéthylène glycol (PEG) de ces composés ; ou n'importe quelle combinaison de ceux-ci.

15. Formulation pharmaceutique selon l'une quelconque des revendications 2 à 8, l'agent biologiquement actif étant l'insuline.

16. Formulation pharmaceutique selon l'une quelconque des revendications 2 à 8, l'agent biologiquement actif étant l'héparine.

17. Formulation pharmaceutique selon l'une quelconque des revendications 2 à 8, l'agent biologiquement actif étant l'héparine non fractionnée.

18. Formulation pharmaceutique selon l'une quelconque des revendications 2 à 8, l'agent biologiquement actif étant l'héparine de bas poids moléculaire.

19. Formulation pharmaceutique selon l'une quelconque des revendications 2 à 18, les particules présentant un enrobage entérique.

20. Unité posologique solide comprenant la formulation pharmaceutique selon l'une quelconque des revendications 2 à 19.

21. Unité posologique solide selon la revendication 20, comprenant en outre un agent délitant.

22. Unité posologique solide selon la revendication 21, l'agent délitant étant un agent super délitant.

23. Unité posologique solide selon la revendication 22, l'agent super délitant étant le glycolate sodique d'amidon ou la croscarmellose sodique.

24. Unité posologique solide selon la revendication 22, l'agent super délitant étant un agent super délitant d'extra particule.

25. Unité posologique solide selon l'une quelconque des revendications 20 à 24, l'unité posologique solide se présentant sous la forme d'un comprimé.

26. Unité posologique solide selon l'une quelconque des revendications 20 à 24, l'unité posologique solide se présentant sous la forme d'une capsule.

27. Formulation pharmaceutique selon l'une quelconque des revendications 2 à 19 pour l'utilisation dans le traitement du diabète chez un mammifère en ayant besoin.

28. Formulation pour l'utilisation selon la revendication 27, le composé agent de libération étant l'acide 4-[(2-hydroxy-4-chloro-benzoyl)-amino]butanoïque ou son sel pharmaceutiquement acceptable.

29. Formulation pharmaceutique selon l'une quelconque des revendications 2 à 19 pour l'utilisation dans le traitement de la tolérance détériorée au glucose, le diabète à un stade précoce, ou le diabète à un stade tardif ou l'atteinte de l'homéostase du glucose chez un être humain en ayant besoin.

30. Formulation pour l'utilisation selon la revendication 29, comprenant l'administration de la formulation pharmaceutique sur une base chronique.

31. Formulation pharmaceutique selon l'une quelconque des revendications 2 à 19 pour l'utilisation dans le traitement d'un patient diabétique humain, comprenant l'administration par voie orale de la formulation pharmaceutique sur une base chronique.
